# EUROPEAN PATENT SPECIFICATION

(11) **EP 4 214 514 B1**
(45) Date of publication and mention of the grant of the patent: **20.05.2026**
(21) Application number: 21786098.0
(22) Date of filing: 20.09.2021
(51) Int. Cl.: G01N 33/66, G01N 33/68

(54) **A METHOD OF EVALUATING SPERM HEALTH AND IDENTIFYING SPERM TO BE USED IN ASSISTED REPRODUCTIVE TECHNOLOGIES**
VERFAHREN ZUR BEURTEILUNG DER SPERMIENGESUNDHEIT UND ZUR IDENTIFIZIERUNG VON SPERMIEN ZUR VERWENDUNG IN UNTERSTÜTZTEN REPRODUKTIONSTECHNOLOGIEN
PROCÉDÉ D'ÉVALUATION DE LA SANTÉ DU SPERME ET D'IDENTIFICATION DU SPERME À UTILISER POUR DES TECHNOLOGIES DE REPRODUCTION ASSISTÉE

(30) Priority: 18.09.2020 GB 202014788
(43) Date of publication of application: 26.07.2023
(73) Proprietor: The Provost, Fellows, Foundation Scholars, and The Other Members of Board, of The College of The Holy and Undivided Trinity of Queen Elizabeth, Dublin 2 (IE)
(72) Inventor: KELLY, Vincent, 2 Dublin (IE); BROWNING, Jill, 2 Dublin (IE); JAGOE, William, 2 Dublin (IE)
(74) Representative: FRKelly
(86) International application number: PCT/EP2021/075820
(87) International publication number: WO 2022/058592

(56) References cited:
- MALLIDIS CON ET AL: "Distribution of the receptor for advanced glycation end products in the human male reproductive tract: prevalence in men with diabetes mellitus", HUMAN REPRODUCTION, vol. 22, no. 8, 22 June 2007 (2007-06-22), GB, pages 2169 - 2177, XP055870478, ISSN: 0268-1161, DOI: 10.1093/humrep/dem156
- KARIMI J. ET AL: "Increased receptor for advanced glycation end products in spermatozoa of diabetic men and its association with sperm nuclear DNA fragmentation : RAGE and sperm nuclear DNA fragmentation", ANDROLOGIA, vol. 44, 15 September 2011 (2011-09-15), DE, pages 280 - 286, XP055870522, ISSN: 0303-4569, DOI: 10.1111/j.1439-0272.2011.01178.x
- UNIT REPRODUCTION ET AL: "From the Human Morphology of Spermatozoa in Infertile Men with and without Varicocele", 1 January 1983 (1983-01-01), XP055870682, Retrieved from the Internet <URL:https://onlinelibrary.wiley.com/doi/pdfdirect/10.1002/j.1939-4640.1983.tb02376.x> [retrieved on 20211208]
- AHADI MAHSA ET AL: "Evaluation of the Standardization in Semen Analysis Performance According to the WHO Protocols Among Laboratories in Tehran, Iran", IRANIAN JOURNAL OF PATHOLOGY, 1 April 2019 (2019-04-01), Tehran, Iran, pages 142 - 147, XP055870726, Retrieved from the Internet <URL:https://www.who.int/docs/default-source/reproductive-health/srhr-documents/infertility/examination-and-processing-of-human-semen-5ed-eng.pdf> [retrieved on 20211208], DOI: 10.30699/ijp.14.2.142

## Description

### Field of the Invention

The present invention relates to a method of evaluating sperm health and identifying sperm to be used in Assisted Reproductive Technologies by providing a biological sample from a patient, preferably a semen sample; measuring the quantitative level of a biomarker in the sample; and evaluating sperm health based on the quantitative level of the biomarker, wherein the biomarker is membrane-bound receptor for advanced glycation end products (mRAGE).

### Background to the Invention

Infertility affects approximately 15% of couples that are of reproductive age and approximately 30-40% of cases can be attributed to male factor infertility. Male infertility can arise from a variety of situations including varicocele, genetic alterations, systemic diseases, and altered semen parameters. However, the single greatest contributory factor to male infertility is sperm damage due to oxidative stress and resultant DNA fragmentation, estimated to account for between 30% and 80% of cases. Not only does DNA fragmentation affect sperm quality, it has also been found to negatively impact embryo quality, implantation rates and miscarriage rates.

Current investigations of male infertility include an evaluation of the physical characteristics of a semen sample (volume, viscosity, pH and colour) and a microscopic evaluation of sperm in a sample (concentration, motility, morphology and vitality). These tests provide a functional and physical read out of the health of the sperm population in a semen sample. Assessment of double-stranded breaks in sperm DNA is recommended to provide additional information about the molecular health of sperm, fertilization rates and DNA integrity. Tests include the terminal deoxynucleotidyl transferase (TdT)-mediated deoxyuridine triphosphate (dUTP)-nick-end labelling (or TUNEL), comet, or sperm chromatin dispersion (SCD) assays. Unfortunately, these tests are often not included in investigations of male infertility as they are expensive, time-consuming, invasive and do not adequately report on sperm health.

Extracellular markers that correlate with the degree of sperm DNA damage and the quality of the sperm would be highly valuable for the identification and removal of damaged sperm prior to assisted reproductive techniques (ART). Significant efforts have been made over the last three decades to identify a more sophisticated marker. Many intracellular markers of DNA fragmentation, oxidative DNA damage, oxidative stress, mitochondrial function and sperm cell death have been identified. However, these require invasive internal labelling procedures leaving the sperm non-viable afterwards. In this study, we evaluate an extracellular marker of sperm cell health.

The Receptor for Advanced Glycation End products (RAGE) is a central signalling molecule of the innate immune system and belongs to a class of cell-surface, pattern recognition receptors encoded at the MHC class III locus. It exists in numerous forms as membrane-bound (full length), soluble or secretory protein. Soluble RAGE (sRAGE) is produced by both proteolytic cleavage of membrane-bound / full length RAGE (mRAGE / fl-RAGE), and alternative mRNA splicing. The soluble isoforms include the extracellular domains but lack the transmembrane and cytoplasmic domains

RAGE is long established as a key contributor to severe chronic pathologies including neurodegeneration, atherosclerosis, cardiovascular diseases, osteoarthritis and diabetes. Under normal conditions, the expression of RAGE is very low. However, during disease, the production of RAGE is highly induced on the cell surface by a number of different ligands such as advanced glycation end products (AGE), high mobility group box-1 (HMGB1), S100/calgranulins, phosphatidylserine, C3. In addition, RAGE expression is further enhanced after immune activation or infection and many RAGE ligands are secreted by monocytes, macrophages, neutrophils and leukocytes which are likely to be crucial for the initiation and propagation of a RAGE-dependent inflammatory response. The location of the membrane-bound receptor at the acrosomal cap of human sperm, which is a direct access point to nuclear DNA within the sperm head, has been implicated in playing a role in mediating and exacerbating nuclear DNA damage.

This study describes mRAGE as a diagnostic biomarker of low-quality sperm. mRAGE expression was analysed on sperm from non-diabetic, non-obese and non-smoking men thereby removing the heightened effects of RAGE and its ligands on sperm health in these conditions. Flow cytometry was used to quantitatively assess mRAGE expression at a cellular level on sperm and computer-assisted sperm analysis (CASA) was used to evaluate sperm motility. By analysing mRAGE as well as mitochondrial membrane potential (MMP), cell permeability, motility, morphology, apoptosis and DNA fragmentation at the cellular level, strongest evidence is provided for a direct relationship.

It was observed that mRAGE expression on sperm correlates with mitochondrial dysfunction, cell death, DNA fragmentation and immotile sperm. In addition, purifying sperm using conventional techniques (density gradient centrifugation and direct swim-up) allowed for the removal of mRAGE expressing spermatozoa. Thus, there remains a need for a reliable, less invasive, extracellular marker of evaluating sperm health. As RAGE is membrane bound, it is also possible to use it as a means to remove the damaged sperm. MALLIDIS CON ET AL., HUMAN REPRODUCTION, vol. 22, no. 8, 22 June 2007 (2007-06-22), pages 2169-2177 and KARIMI J. ET AL., ANDROLOGIA, vol. 44, 15 September 2011, p. 280-286 already disclose methods involving determination of sperm health in diabetic men but here all forms of RAGE are determined but not specifically mRAGE.

### Summary of the Invention

According to a first aspect of the present invention, there is provided a method of evaluating sperm health; the method comprising the steps of:
(a) providing a biological sample from a patient;
(b) measuring the quantitative or qualitative level of a biomarker in the sample; and
(c) evaluating sperm health based on the quantitative or qualitative level of the biomarker, wherein the biomarker is membrane-bound receptor for advanced glycation end products (mRAGE).

Optionally, the method of evaluating sperm health is an in vitro method.

Optionally, mRAGE is bound to a membrane of a cell.

Optionally, mRAGE is bound to the membrane of a sperm.

Optionally, mRAGE is bound to the membrane of the sperm head.

Optionally, mRAGE is bound to the membrane of the equatorial regions of the sperm head.

Optionally, mRAGE is a transmembrane receptor.

Optionally, mRAGE comprises three extracellular domains.

Optionally, mRAGE comprises three extracellular domains - a V-type Ig domain and two C-type Ig domains (C1 and C2).

Optionally, mRAGE comprises three extracellular domains - a V-type Ig domain and two C-type Ig domains (C1 and C2); and a transmembrane domain.

Optionally, mRAGE comprises three extracellular domains, a V-type Ig domain and two C-type Ig domains (C1 and C2); a transmembrane domain; and a cytoplasmic tail.

Optionally, mRAGE is full-length RAGE (fl-RAGE).

Preferably, the biological sample is a semen sample.

Optionally, the biomarker is a gene. Optionally, the biomarker is a nucleic acid. Optionally, the biomarker is a deoxyribonucleic acid. Optionally, the biomarker is a ribonucleic acid. Optionally, the biomarker is a protein. Optionally, the biomarker is a peptide.

Optionally, the biomarker for evaluating sperm health is a gene having a GenBank Accession Version Number AQY76652
Preferably, the biomarker for evaluating sperm health is a protein having a UniProt ID Q15109
Preferably, the measuring step (b) comprises measuring the quantitative level of mRAGE on sperm cells from a semen sample.

Optionally, the measuring step (b) comprises measuring the quantitative level of mRAGE on sperm cells from a semen sample by any quantitative analytical technique.

Optionally, the measuring step (b) comprises measuring the quantitative or qualitative level of mRAGE on sperm cells from a semen sample by a cell-based assay.

Optionally, the measuring step (b) comprises measuring the quantitative or qualitative level of mRAGE on sperm cells from a semen sample by flow cytometry.

Optionally, the measuring step (b) comprises measuring the quantitative or qualitative level of mRAGE on sperm cells from a semen sample and is determined using a RAGE antibody.

Optionally, the measuring step (b) comprises measuring the quantitative or qualitative level of mRAGE on sperm cells from a semen sample and is determined using a sRAGE antibody.

Optionally, the measuring step (b) comprises measuring the quantitative or qualitative level of mRAGE on sperm cells from a semen sample and is determined using a mRAGE antibody.

Optionally, the measuring step (b) comprises measuring the quantitative or qualitative level of mRAGE on sperm cells from a semen sample and is determined using a mouse anti-human mRAGE antibody.

Optionally, the measuring step (b) comprises measuring the quantitative or qualitative level of mRAGE on sperm cells from a semen sample and is determined using a rabbit anti-human mRAGE antibody.

Optionally, the measuring step (b) comprises measuring the quantitative or qualitative level of mRAGE on sperm cells from a semen sample and is determined using a rat anti-human mRAGE antibody.

Optionally, the measuring step (b) comprises measuring the quantitative or qualitative level of mRAGE on sperm cells from a semen sample and is determined using a sheep anti-human mRAGE antibody.

Optionally, the measuring step (b) comprises measuring the quantitative or qualitative level of mRAGE on sperm cells from a semen sample and is determined using a monoclonal rabbit anti-human mRAGE antibody.

Optionally, the measuring step (b) comprises measuring the quantitative or qualitative level of mRAGE on sperm cells from a semen sample and is determined using a monoclonal rat anti-human mRAGE antibody.

Optionally, the measuring step (b) comprises measuring the quantitative or qualitative level of mRAGE on sperm cells from a semen sample and is determined using a monoclonal sheep anti-human mRAGE antibody.

Optionally, the measuring step (b) comprises measuring the quantitative or qualitative level of mRAGE on sperm cells from a semen sample and is determined using a monoclonal mouse anti-human mRAGE antibody.

Optionally, the measuring step (b) comprises measuring the quantitative or qualitative level of mRAGE on sperm cells from a semen sample and is determined using the monoclonal mouse anti-human mRAGE antibody - AF647.

Optionally, the measuring step (b) comprises measuring the quantitative or qualitative level of mRAGE on sperm cells from a semen sample and is determined using DNA aptamers.

Optionally, the measuring step (b) comprises measuring the quantitative or qualitative level of mRAGE on sperm cells from a semen sample and is determined using RNA aptamers.

Optionally, the measuring step (b) comprises measuring the quantitative or qualitative level of mRAGE on sperm cells from a semen sample and is determined using peptide aptamers.

Optionally, the evaluating step (c) comprises evaluating sperm health based on the quantitative or qualitative level of mRAGE on sperm cells from a semen sample
Optionally, the evaluating step (c) comprises evaluating sperm health based on the quantitative or qualitative level of mRAGE on sperm cells from a semen sample, wherein the presence of mRAGE is defined as mRAGE positive.

Optionally, the evaluating step (c) comprises evaluating the presence or absence of quantifiable levels of mRAGE on sperm cells from a semen sample to define the patient as mRAGE negative or mRAGE positive.

Optionally, the presence of quantifiable levels of mRAGE on sperm cells from the semen sample defines the patient as mRAGE positive and wherein the absence of quantifiable levels of mRAGE on sperm cells from the semen sample defines the patient as mRAGE negative.

Optionally, the evaluating step (c) comprises comparing the amount of sperm expressing quantifiable levels of mRAGE to the amount of sperm not expressing quantifiable levels of mRAGE.

Optionally, the amount of sperm expressing quantifiable levels of mRAGE deviates from the amount of sperm not expressing quantifiable levels of mRAGE by at least 1-fold.

Optionally, the amount of sperm expressing quantifiable levels of mRAGE deviates from the amount of sperm not expressing quantifiable levels of mRAGE by at least 2-fold.

Optionally, the amount of sperm expressing quantifiable levels of mRAGE deviates from the amount of sperm not expressing quantifiable levels of mRAGE by at least 3-fold.

Optionally, the amount of sperm expressing quantifiable levels of mRAGE deviates from the amount of sperm not expressing quantifiable levels of mRAGE by at least 4-fold.

Optionally, the amount of sperm expressing quantifiable levels of mRAGE deviates from the amount of sperm not expressing quantifiable levels of mRAGE by at least 5-fold.

Optionally, the amount of sperm expressing quantifiable levels of mRAGE deviates from the amount of sperm not expressing quantifiable levels of mRAGE by at least 6-fold.

Optionally, the amount of sperm expressing quantifiable levels of mRAGE deviates from the amount of sperm not expressing quantifiable levels of mRAGE by at least 7-fold.

Optionally, the amount of sperm expressing quantifiable levels of mRAGE deviates from the amount of sperm not expressing quantifiable levels of mRAGE by at least 8-fold.

Optionally, the amount of sperm expressing quantifiable levels of mRAGE deviates from the amount of sperm not expressing quantifiable levels of mRAGE by at least 9-fold.

Optionally, the amount of sperm expressing quantifiable levels of mRAGE deviates from the amount of sperm not expressing quantifiable levels of mRAGE by at least 10-fold.

Optionally, the evaluating step (c) comprises evaluating the percentage of sperm expressing quantifiable levels of mRAGE on sperm cells from a semen sample.

Optionally, the evaluating step (c) comprises evaluating the percentage of sperm expressing quantifiable levels of mRAGE on sperm cells from a semen sample to define the patient as mRAGE negative or mRAGE positive.

Optionally, the evaluating step (c) comprises evaluating the percentage of sperm expressing quantifiable levels of mRAGE on sperm cells from a semen sample, wherein mRAGE negative is defined as a Mean Fluorescent Intensity (MFI; geometric mean) value that is lower than 10-fold compared to the mRAGE positive sperm cells.

Optionally, the evaluating step (c) comprises evaluating the percentage of sperm expressing qualifiable levels of mRAGE on sperm cells from a semen sample, wherein mRAGE positive is defined as a Mean Fluorescent Intensity (MFI; geometric mean) value that is at least 10-fold higher compared to the mRAGE negative sperm cells.

Optionally, the evaluating step (c) comprises evaluating the percentage of sperm expressing quantifiable levels of mRAGE on sperm cells from a semen sample, wherein mRAGE positive is defined as a Mean Fluorescent Intensity (MFI; geometric mean) value that is at least 20-fold higher compared to the mRAGE negative sperm cells.

Optionally, the evaluating step (c) comprises evaluating the percentage of sperm expressing quantifiable levels of mRAGE on sperm cells from a semen sample, wherein mRAGE positive is defined as a Mean Fluorescent Intensity (MFI; geometric mean) value that is at least 30-fold higher compared to the mRAGE negative sperm cells.

Optionally, a Mean Fluorescent Intensity value expressing quantifiable levels of mRAGE deviates from a Mean Fluorescent Intensity value not expressing quantifiable levels of mRAGE by less than 10-fold.

Optionally, a Mean Fluorescent Intensity value expressing quantifiable levels of mRAGE deviates from a Mean Fluorescent Intensity value not expressing quantifiable levels of mRAGE by at least 10-fold.

Optionally, the evaluating step (c) comprises evaluating sperm health based on the quantitative or qualitative level of mRAGE on sperm cells from a semen sample, wherein mRAGE positive is indicative of poor sperm health.

Optionally, the evaluating step (c) comprises evaluating sperm health based on the quantitative or qualitative level of mRAGE on sperm cells from a semen sample, wherein mRAGE negative is indicative of healthy, fertile sperm.

Further optionally, the evaluating step (c) comprises evaluating sperm health based on the quantitative or qualitative level of mRAGE on sperm cells from a semen sample, wherein the mRAGE positive is indicative of abnormal sperm.

Further optionally, the evaluating step (c) comprises evaluating sperm health based on the quantitative or qualitative level of mRAGE on sperm cells from a semen sample, wherein the mRAGE negative is indicative of normal sperm.

Further optionally, the evaluating step (c) comprises evaluating sperm health based on the quantitative or qualitative level of mRAGE on sperm cells from a semen sample, wherein the mRAGE negative is indicative of good sperm health.

Further optionally, the evaluating step (c) comprises evaluating sperm health based on the quantitative or qualitative level of mRAGE on sperm cells from a semen sample, wherein the mRAGE negative is indicative of one or more of normal sperm and good sperm health.

Optionally, the evaluating step (c) comprises evaluating sperm health based on the quantitative or qualitative level of mRAGE on sperm cells from a semen sample, wherein the mRAGE positive is indicative of one or more of poor sperm health and abnormal sperm.

Further optionally, the evaluating step (c) comprises evaluating sperm health based on the quantitative or qualitative level of mRAGE on sperm cells from a semen sample, wherein the mRAGE positive is indicative of poor sperm motility.

Optionally, the evaluating step (c) comprises evaluating sperm health based on the quantitative or qualitative level of mRAGE on sperm cells from a semen sample, wherein mRAGE positive is indicative of one or more of poor sperm health, abnormal sperm and poor sperm motility.

Further optionally, the evaluating step (c) comprises evaluating sperm health based on the quantitative or qualitative level of mRAGE on sperm cells from a semen sample, wherein the mRAGE positive is indicative of infertility.

Optionally, the evaluating step (c) comprises evaluating sperm health based on the quantitative or qualitative level of mRAGE on sperm cells from a semen sample, wherein the mRAGE positive is indicative of one or more of poor sperm health, abnormal sperm, poor sperm motility and infertility.

Optionally, in the method of evaluating sperm health, the patient is a mammal.

Optionally, in the method of evaluating sperm health, the patient is non-human
Optionally, in the method of evaluating sperm health, the patient is non-human, such as bovine, equine, canine, feline, porcine, ovine and ursidae animals.

Preferably, in the method of evaluating sperm health, the patient is human.

Optionally, in the method of evaluating sperm health, the patient is non-diabetic human.

Optionally, in the method of evaluating sperm health, the patient is non-diabetic, non-smoking human.

Optionally, in the method of evaluating sperm health, the patient is non-diabetic, non-smoking, non-obese human.

Optionally, in the method of evaluating sperm health, the patient is non-diabetic, non-smoking, non-obese (BMI < 29.9) human.

Optionally, in the method of evaluating sperm health, the patient is non-diabetic, non-smoking, non-obese (BMI < 29.9) man.

Optionally, the method of evaluating sperm health further comprises assessing sperm motility.

Optionally, the method of evaluating sperm health further comprises assessing sperm motility, wherein the sperm are categorised as immotile (IM), non-progressively motile (NP) or progressively motile (PR).

Optionally, the method of evaluating sperm health further comprises assessing sperm motility, wherein sperm categorised as IM do not move.

Optionally, the method of evaluating sperm health further comprises assessing sperm motility, wherein sperm categorised as IM do not move, which is indicative of poor sperm health.

Optionally, the method of evaluating sperm health further comprises assessing sperm motility, wherein sperm categorised as IM do not move, which is indicative of poor sperm health and poor sperm motility.

Optionally, the method of evaluating sperm health further comprises assessing sperm motility, wherein sperm categorised as IM do not move, as defined by the World Health Organisation (WHO) laboratory manual for the examination and processing of human semen (fifth edition, 2010).

Optionally, the method of evaluating sperm health further comprises assessing sperm motility, wherein sperm categorised as IM do not move, which is indicative of poor sperm health, as defined by the World Health Organisation (WHO) laboratory manual for the examination and processing of human semen (fifth edition, 2010).

Optionally, the method of evaluating sperm health further comprises assessing sperm motility, wherein sperm categorised as NP move, but do not move in a substantially linear direction.

Optionally, the method of evaluating sperm health further comprises assessing sperm motility, wherein sperm categorised as NP move, but do not move in a substantially linear direction, as defined by the World Health Organisation (WHO) laboratory manual for the examination and processing of human semen (fifth edition, 2010).

Optionally, the method of evaluating sperm health further comprises assessing sperm motility, wherein sperm categorised as PR move in a substantially linear direction.

Optionally, the method of evaluating sperm health further comprises assessing sperm motility, wherein sperm categorised as PR move in a substantially linear direction, as defined by the World Health Organisation (WHO) laboratory manual for the examination and processing of human semen (fifth edition, 2010).

Optionally, the method of evaluating sperm health further comprises assessing sperm motility, wherein at least 10% of sperm categorised as PR in a semen sample is considered normal.

Optionally, the method of evaluating sperm health further comprises assessing sperm motility, wherein at least 20% of sperm categorised as PR in a semen sample is considered normal.

Optionally, the method of evaluating sperm health further comprises assessing sperm motility, wherein at least 30% of sperm categorised as PR in a semen sample is considered normal.

Optionally, the method of evaluating sperm health further comprises assessing sperm motility, wherein at least 32% of sperm categorised as PR in a semen sample is considered normal.

Optionally, the method of evaluating sperm health further comprises assessing sperm motility, wherein at least 32% of sperm categorised as PR in a semen sample is considered normal, as defined by the World Health Organisation (WHO) laboratory manual for the examination and processing of human semen (fifth edition, 2010).

Optionally, the method of evaluating sperm health further comprises assessing sperm motility, wherein at least 10% of a combination of sperm categorised as NP and sperm categorised as PR in a semen sample is considered normal.

Optionally, the method of evaluating sperm health further comprises assessing sperm motility, wherein at least 20% of a combination of sperm categorised as NP and sperm categorised as PR in a semen sample is considered normal.

Optionally, the method of evaluating sperm health further comprises assessing sperm motility, wherein at least 30% of a combination of sperm categorised as NP and sperm categorised as PR in a semen sample is considered normal.

Optionally, the method of evaluating sperm health further comprises assessing sperm motility, wherein at least 40% of a combination of sperm categorised as NP and sperm categorised as PR in a semen sample is considered normal.

Optionally, the method of evaluating sperm health further comprises assessing sperm motility, wherein at least 40% of a combination of sperm categorised as NP and sperm categorised as PR in a semen sample is considered normal, as defined by the World Health Organisation (WHO) laboratory manual for the examination and processing of human semen (fifth edition, 2010).

Optionally, the method of evaluating sperm health further comprises assessing sperm motility, wherein the percentage of mRAGE positive sperm is inversely correlated to the percentage of PR sperm; defined as sperm with a space gain of ≥5 µm s⁻¹.

Optionally, the method of evaluating sperm health further comprises assessing sperm morphology.

Optionally, the method of evaluating sperm health further comprises assessing sperm morphology, wherein the sperm are classified as normal or abnormal.

Optionally, the method of evaluating sperm health further comprises assessing sperm morphology, wherein the sperm are classified as normal or abnormal, as defined by the World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape.

Optionally, the method of evaluating sperm health further comprises assessing sperm morphology, wherein sperm are classified as normal when the sperm head is smooth, regularly contoured and substantially oval in shape, as defined by the World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape; and there is a well-defined acrosomal region comprising 40-70% of the sperm head area.

Optionally, the method of evaluating sperm health further comprises assessing sperm morphology, wherein sperm are classified as normal when the sperm head is smooth, regularly contoured and substantially oval in shape; and there is a well-defined acrosomal region comprising 40-70% of the sperm head area, as defined by the World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; and the acrosomal region contains no large vacuoles, and not more than two small vacuoles, which does not occupy more than 20% of the sperm head.

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; and the acrosomal region contains no large vacuoles, and not more than two small vacuoles, which does not occupy more than 20% of the sperm head, as defined by the World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; and the midpiece of the sperm is slender, regular and substantially the same length as the sperm head.

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; and the midpiece of the sperm is slender, regular and substantially the same length as the sperm head, as by the defined World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; the midpiece of the sperm is slender, regular and substantially the same length as the sperm head; and the major axis of the midpiece of the sperm is aligned with the major axis of the sperm head.

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; the midpiece of the sperm is slender, regular and substantially the same length as the sperm head; and the major axis of the midpiece of the sperm is aligned with the major axis of the sperm head, as defined by the World Health Organisation (WHO) laboratory manual for the examination and processing of human semen (fifth edition, 2010).

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; the midpiece of the sperm is slender, regular and substantially the same length as the sperm head; the major axis of the midpiece of the sperm is aligned with the major axis of the sperm head; and the principal piece has a uniform calibre along its length, is thinner than the midpiece, and is approximately 45 micrometres long.

Optionally, the method of evaluating sperm health further comprises assessing sperm morphology, wherein sperm are classified as normal when the sperm head is smooth, regularly contoured and substantially oval in shape; there is a well-defined acrosomal region comprising 40-70% of the sperm head area; the acrosomal region contains no large vacuoles, and not more than two small vacuoles, which does not occupy more than 20% of the sperm head; the midpiece of the sperm is slender, regular and substantially the same length as the sperm head; the major axis of the midpiece of the sperm is aligned with the major axis of the sperm head; and the principal piece has a uniform calibre along its length, is thinner than the midpiece, and is approximately 45 micrometres long, as defined by the World Health Organisation (WHO) laboratory manual for the examination and processing of human semen (fifth edition, 2010).

Optionally, the method of evaluating sperm health further comprises assessing sperm morphology, wherein sperm are classified as abnormal when there is deviation from normal.

Optionally, the method of evaluating sperm health further comprises assessing sperm morphology, wherein sperm are classified as abnormal when there is deviation from normal, as defined by the World Health Organisation (WHO) laboratory manual for the examination and processing of human semen (fifth edition, 2010).

Optionally, the method of evaluating sperm health further comprises assessing sperm morphology, wherein sperm are classified as abnormal when there is deviation from normal, which is indicative of poor sperm health.

Optionally, the method of evaluating sperm health further comprises assessing sperm morphology, wherein sperm are classified as abnormal when there is deviation from normal, which is indicative of poor sperm health, as defined by the World Health Organisation (WHO) laboratory manual for the examination and processing of human semen (fifth edition, 2010).

Optionally, the method of evaluating sperm health further comprises a step of assessing mitochondrial membrane potential (MMP).

Optionally, the method of evaluating sperm health further comprises a step of assessing MMP, wherein MMP is assessed by staining with Mitotracker CMX Ros.

Optionally, the method of evaluating sperm health further comprises a step of assessing MMP, wherein MMP is assessed by staining with Mitotracker CMX Ros, wherein Mitotracker CMX Ros does not stain cells with low MMP.

Optionally, the method of evaluating sperm health further comprises a step of assessing MMP, wherein MMP is assessed by staining with Mitotracker CMX Ros, wherein Mitotracker CMX Ros does not stain cells with low MMP, which is indicative of poor sperm health.

Optionally, the method of evaluating sperm health further comprises a step of assessing MMP, wherein MMP is assessed by staining with Mitotracker CMX Ros, wherein Mitotracker CMX Ros does not stain cells with low MMP; as defined as a Mean Fluorescent Intensity (MFI; geometric mean) value that is 10-fold, or lower, than the mRAGE negative sperm cells, which is indicative of poor sperm health.

Optionally, the method of evaluating sperm health further comprises a step of assessing MMP, wherein MMP is assessed by staining with Mitotracker CMX Ros, wherein Mitotracker CMX Ros stains cells with high MMP.

Optionally, the method of evaluating sperm health further comprises a step of assessing MMP, wherein MMP is assessed by staining with Mitotracker CMX Ros, wherein Mitotracker CMX Ros stains cells with high MMP, which is indicative of good sperm health.

Optionally, the method of evaluating sperm health further comprises a step of assessing MMP, wherein MMP is assessed by staining with Mitotracker CMX Ros and an increase in sperm with low MMP is present in mRAGE positive sperm.

Optionally, the method of evaluating sperm health further comprises a step of assessing MMP, wherein MMP is assessed by staining with Mitotracker CMX Ros and an increase in sperm with low MMP is present in mRAGE positive sperm, which is indicative of poor sperm health.

Optionally, the method of evaluating sperm health further comprises a step of assessing MMP, wherein MMP is assessed by staining with Mitotracker CMX Ros and an increase in sperm with high MMP is present in mRAGE negative sperm.

Optionally, the method of evaluating sperm health further comprises a step of assessing MMP, wherein MMP is assessed by staining with Mitotracker CMX Ros and an increase in sperm with high MMP is present in mRAGE negative sperm, which is indicative of good sperm health.

Optionally, the method of evaluating sperm health further comprises a step of assessing MMP, wherein MMP is assessed by staining with Mitotracker CMX Ros and an increase in sperm with high MMP is present in mRAGE negative sperm compared to mRAGE positive sperm.

Optionally, the method of evaluating sperm health further comprises a step of assessing MMP, wherein MMP is assessed by staining with Mitotracker CMX Ros and at least a 2-fold increase in sperm with high MMP is present in mRAGE negative sperm compared to RAGE positive sperm.

Optionally, the method of evaluating sperm health further comprises a step of assessing MMP, wherein MMP is assessed by staining with Mitotracker CMX Ros and at least a 2-fold increase in sperm with high MMP is present in mRAGE negative sperm compared to RAGE positive sperm, which is indicative of good sperm health.

Optionally, the method of evaluating sperm health further comprises a step of assessing MMP, wherein MMP is assessed by staining with Mitotracker CMX Ros and at least a 5-fold increase in sperm with high MMP is present in mRAGE negative sperm compared to RAGE positive sperm.

Optionally, the method of evaluating sperm health further comprises a step of assessing MMP, wherein MMP is assessed by staining with Mitotracker CMX Ros and at least a 5-fold increase in sperm with high MMP is present in mRAGE negative sperm compared to RAGE positive sperm, which is indicative of good sperm health.

Optionally, the method of evaluating sperm health further comprises a step of assessing MMP, wherein MMP is assessed by staining with Mitotracker CMX Ros and at least a 10-fold increase in sperm with high MMP is present in mRAGE negative sperm compared to mRAGE positive sperm.

Optionally, the method of evaluating sperm health further comprises a step of assessing MMP, wherein MMP is assessed by staining with Mitotracker CMX Ros and at least a 10-fold increase in sperm with high MMP is present in mRAGE negative sperm compared to mRAGE positive sperm, which is indicative of good sperm health.

Optionally, the method of evaluating sperm health further comprises a step of assessing MMP, wherein MMP is assessed by staining with Mitotracker CMX Ros and at least a 2-fold decrease in sperm with high MMP is present in mRAGE positive sperm compared to RAGE negative sperm.

Optionally, the method of evaluating sperm health further comprises a step of assessing MMP, wherein MMP is assessed by staining with Mitotracker CMX Ros and at least a 2-fold decrease in sperm with high MMP is present in mRAGE positive sperm compared to RAGE negative sperm, which is indicative of poor sperm health.

Optionally, the method of evaluating sperm health further comprises a step of assessing MMP, wherein MMP is assessed by staining with Mitotracker CMX Ros and at least a 5-fold decrease in sperm with high MMP is present in mRAGE positive sperm compared to RAGE negative sperm.

Optionally, the method of evaluating sperm health further comprises a step of assessing MMP, wherein MMP is assessed by staining with Mitotracker CMX Ros and at least a 5-fold decrease in sperm with high MMP is present in mRAGE positive sperm compared to RAGE negative sperm, which is indicative of poor sperm health.

Optionally, the method of evaluating sperm health further comprises a step of assessing MMP, wherein MMP is assessed by staining with Mitotracker CMX Ros and at least a 10-fold decrease in sperm with high MMP is present in mRAGE positive sperm compared to mRAGE negative sperm, which is indicative of poor sperm health.

Optionally, the method of evaluating sperm health further comprises a step of assessing MMP, wherein MMP is assessed by staining with Mitotracker CMX Ros and at least a 10-fold decrease in sperm with high MMP is present in mRAGE positive sperm compared to mRAGE negative sperm.

Optionally, quantifiable levels of MMP is indicative of mRAGE negative sperm and the absence of quantifiable levels of MMP is indicative of mRAGE positive sperm.

Optionally, the method of evaluating sperm health further comprises the step of assessing cell permeability.

Optionally, the method of evaluating sperm health further comprises assessing cell permeability, wherein cell permeability is assessed by staining with DAPI.

Optionally, the method of evaluating sperm health further comprises assessing cell permeability, wherein cell permeability is assessed by staining with DAPI, and positive staining by DAPI represents non-viable cells.

Optionally, the method of evaluating sperm health further comprises assessing cell permeability, wherein cell permeability is assessed by staining with DAPI, and positive staining by DAPI represents non-viable cells, wherein an increase in non-viable cells is present in mRAGE positive sperm.

Optionally, the method of evaluating sperm health further comprises assessing cell permeability, wherein cell permeability is assessed staining with DAPI, and positive staining by DAPI represents non-viable cells, wherein an increase in non-viable cells is present in mRAGE positive sperm, which is indicative of poor sperm health.

Optionally, the method of evaluating sperm health further comprises assessing cell permeability, wherein cell permeability is assessed staining with DAPI, and positive staining by DAPI represents non-viable cells, wherein an increase in non-viable cells is present in mRAGE positive sperm compared to mRAGE negative sperm, which is indicative of poor sperm health.

Optionally, the method of evaluating sperm health further comprises assessing cell permeability, wherein cell permeability is assessed staining with DAPI, and positive staining by DAPI represents non-viable cells, wherein at least a 2-fold increase in non-viable cells is present in mRAGE positive sperm compared to mRAGE negative sperm, which is indicative of poor sperm health.

Optionally, the method of evaluating sperm health further comprises assessing cell permeability, wherein cell permeability is assessed staining with DAPI, and positive staining by DAPI represents non-viable cells, wherein at least a 3-fold increase in non-viable cells is present in mRAGE positive sperm compared to mRAGE negative sperm, which is indicative of poor sperm health.

Optionally, at least a 2-fold decrease in non-permeable cells is present in mRAGE positive sperm compared to mRAGE negative sperm, which is indicative of poor sperm health

Optionally, the method of evaluating sperm health further comprises the step of assessing apoptosis.

Optionally, the method of evaluating sperm health further comprises assessing apoptosis, wherein apoptosis is assessed by staining with Annexin V.

Optionally, the method of evaluating sperm health further comprises assessing apoptosis, wherein apoptosis is assessed by staining with Annexin V and positive staining with Annexin V is indicative of poor sperm health.

Optionally, the method of evaluating sperm health further comprises assessing apoptosis, wherein apoptosis is assessed by staining with Annexin V in combination with DAPI.

Optionally, the method of evaluating sperm health further comprises assessing apoptosis, wherein apoptosis is assessed by staining with Annexin V in combination with DAPI to allow for the simultaneous assessment of apoptosis and necrosis.

Optionally, the method of evaluating sperm health further comprises assessing apoptosis, wherein apoptosis is assessed by staining with Annexin V in combination with DAPI to allow for the simultaneous assessment of apoptosis and necrosis, and positive staining with Annexin V in combination with DAPI is indicative of poor sperm health.

Optionally, the method of evaluating sperm health further comprises assessing apoptosis, wherein apoptosis is assessed by staining with Annexin V in combination with DAPI to allow for the simultaneous assessment of apoptosis and necrosis, and an increase in apoptosis and necrosis is present in mRAGE positive sperm.

Optionally, the method of evaluating sperm health further comprises assessing apoptosis, wherein apoptosis is assessed by staining with Annexin V in combination with DAPI to allow for the simultaneous assessment of apoptosis and necrosis, and an increase in apoptosis and necrosis is present in mRAGE positive sperm, which is indicative of poor sperm health.

Optionally, the method of evaluating sperm health further comprises assessing apoptosis, wherein apoptosis is assessed by staining with Annexin V in combination with DAPI to allow for the simultaneous assessment of apoptosis and necrosis, and an increase in apoptosis and necrosis is present in mRAGE positive sperm compared to mRAGE negative sperm, which is indicative of poor sperm health.

Optionally, the method of evaluating sperm health further comprises assessing apoptosis, wherein apoptosis is assessed by staining with Annexin V in combination with DAPI to allow for the simultaneous assessment of apoptosis and necrosis, and at least a 2-fold increase in apoptosis and necrosis is present in mRAGE positive sperm compared to mRAGE negative sperm, which is indicative of poor sperm health.

Optionally, the method of evaluating sperm health further comprises assessing apoptosis, wherein apoptosis is evaluated by staining with Annexin V in combination with DAPI to allow for the simultaneous assessment of apoptosis and necrosis, and at least a 4-fold increase in apoptosis and necrosis is present in mRAGE positive sperm compared to mRAGE negative sperm, which is indicative of poor sperm health.

Optionally, the method of evaluating sperm health further comprises assessing apoptosis, wherein apoptosis is assessed by staining with Annexin V in combination with DAPI to allow for the simultaneous assessment of apoptosis and necrosis, and at least a 4-fold increase in apoptosis and necrosis is present in mRAGE positive sperm compared to mRAGE negative sperm, which is indicative of poor sperm health.

Optionally, the method of evaluating sperm health further comprises the step of assessing DNA fragmentation.

Optionally, the method of evaluating sperm health further comprises assessing DNA fragmentation using the ApoBrdU-terminal deoxynucleotidyl transferase dUTP nick-end labelling (TUNEL) assay.

Optionally, the method of evaluating sperm health further comprises assessing DNA fragmentation using the ApoBrdU-terminal deoxynucleotidyl transferase dUTP nick-end labelling (TUNEL) assay, wherein an increase in DNA fragmentation is present in mRAGE positive sperm, which is indicative of poor sperm health.

Optionally, the method of evaluating sperm health further comprises assessing DNA fragmentation using the ApoBrdU-terminal deoxynucleotidyl transferase dUTP nick-end labelling (TUNEL) assay, wherein an increase in DNA fragmentation is present in mRAGE positive sperm compared to mRAGE negative sperm, which is indicative of poor sperm health.

Optionally, the method of evaluating sperm health further comprises assessing DNA fragmentation using the ApoBrdU-terminal deoxynucleotidyl transferase dUTP nick-end labelling (TUNEL) assay, wherein at least a 2-fold increase in DNA fragmentation is present in mRAGE positive sperm compared to mRAGE negative sperm, which is indicative of poor sperm health.

Optionally, the method of evaluating sperm health further comprises assessing DNA fragmentation using the ApoBrdU-terminal deoxynucleotidyl transferase dUTP nick-end labelling (TUNEL) assay, wherein at least a 3-fold increase in DNA fragmentation is present in mRAGE positive sperm compared to mRAGE negative sperm, which is indicative of poor sperm health.

Optionally, the method of evaluating sperm health further comprises the step of identifying sperm that can be used in assisted reproductive technologies (ART).

Optionally, the method of evaluating sperm health further comprises identifying sperm that can be used in assisted reproductive technologies (ART), including intrauterine insemination (IUI).

Optionally, the method of evaluating sperm health further comprises identifying sperm that can be used in assisted reproductive technologies (ART), including any one or more of intrauterine insemination (IUI) and intracytoplasmic injection (ICSI).

Optionally, the method of evaluating sperm health further comprises identifying sperm that can be used in assisted reproductive technologies (ART), including any one or more of intrauterine insemination (IUI), intracytoplasmic injection (ICSI) and *in vitro* fertilisation (IVF).

Optionally, the method of evaluating sperm health further comprises identifying sperm that can be used in assisted reproductive technologies (ART), including any one or more of intrauterine insemination (IUI), intracytoplasmic injection (ICSI), *in vitro* fertilisation (IVF) and artificial insemination (Al).

Optionally, the method of evaluating sperm health further comprises identifying sperm that can be used in assisted reproductive technologies (ART), wherein the semen sample is processed.

Optionally, the method of evaluating sperm health further comprises identifying sperm that can be used in assisted reproductive technologies (ART), wherein the semen sample is processed using separation techniques.

Optionally, the method of evaluating sperm health further comprises identifying sperm that can be used in assisted reproductive technologies (ART), wherein the semen sample is processed using separation techniques, including density gradient centrifugation (DGC).

Optionally, the method of evaluating sperm health further comprises identifying sperm that can be used in assisted reproductive technologies (ART), wherein the semen sample is processed using separation techniques, including the sperm swim-up method.

Optionally, the method of evaluating sperm health further comprises identifying sperm that can be used in assisted reproductive technologies (ART), wherein the semen sample is processed using separation techniques, including the sperm swim-up method, as defined World Health Organisation (WHO) laboratory manual for the examination and processing of human semen (fifth edition, 2010).

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method separates sperm based on their densities.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method separates sperm based on their densities as morphological normal sperm have a higher density than abnormal sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method separates sperm based on their densities as morphological normal sperm have a higher density than abnormal sperm; and the swim-up method relies on sperm motility.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method separates sperm based on their densities as morphological normal sperm have a higher density than abnormal sperm; and the swim-up method relies on sperm motility to separate PR sperm from IM sperm and NP sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in a decrease in mRAGE positive sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the swim-up method results in a decrease in mRAGE positive sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in a decrease in mRAGE positive sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in a decrease in mRAGE positive sperm, which is indicative of good sperm health.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in at least a 2-fold decrease in mRAGE positive sperm compared to mRAGE negative sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 2-fold decrease in mRAGE positive sperm compared to mRAGE negative sperm and the swim-up method results in at least a 2-fold decrease in mRAGE positive sperm compared to mRAGE negative sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 2-fold decrease in mRAGE positive processed sperm compared to mRAGE negative processed sperm and the swim-up method results in at least a 2-fold decrease in mRAGE positive processed sperm compared to mRAGE negative processed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 3-fold decrease in mRAGE positive processed sperm compared to mRAGE negative processed sperm and the swim-up method results in at least a 3-fold decrease in mRAGE positive processed sperm compared to mRAGE negative processed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 4-fold decrease in mRAGE positive processed sperm compared to mRAGE negative processed sperm and the swim-up method results in at least a 4-fold decrease in mRAGE positive processed sperm compared to mRAGE negative processed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 4-fold decrease in mRAGE positive processed sperm compared to mRAGE negative processed sperm and the swim-up method results in at least a 5-fold decrease in mRAGE positive processed sperm compared to mRAGE negative processed sperm.

Optionally, the method of evaluating sperm health further comprises identifying sperm that can be used in assisted reproductive technologies (ART), wherein the semen sample is processed using separation techniques, including any one or more density gradient centrifugation (DGC) and the sperm swim-up method, wherein the DCG method results in an increase in PR sperm.

Optionally, the method of evaluating sperm health further comprises identifying sperm can be used in assisted reproductive technologies (ART), wherein the semen sample is processed using separation techniques, including any one or more density gradient centrifugation (DGC) and the sperm swim-up method, wherein the swim up method results in an increase in PR sperm.

Optionally, the method of evaluating sperm health further comprises identifying sperm that can be used in assisted reproductive technologies (ART), wherein the semen sample is processed using separation techniques, including any one or more density gradient centrifugation (DGC) and the sperm swim-up method, wherein the DGC method and the swim up method results in an increase in PR sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim up method results in at least a 2-fold increase in PR sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 2-fold increase in PR processed sperm compared to unprocessed sperm and the swim-up method results in at least a 2-fold increase in PR processed sperm compared to unprocessed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 3-fold increase in PR processed sperm compared to unprocessed sperm and the swim-up method results in at least a 3-fold increase in PR processed sperm compared to unprocessed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in at least a 3.5-fold increase in PR processed sperm compared to unprocessed sperm and the swim-up method results in at least a 3.4-fold increase in PR processed sperm compared to unprocessed sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in sperm with a decrease in cell permeability.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the swim-up method results in sperm with a decrease in cell permeability.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC and the swim-up method results sperm with a decrease in cell permeability.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in sperm with at least a 2-fold decrease in cell permeability.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in sperm with at least a 2.75-fold decrease in cell permeability.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method results in a decrease in apoptotic sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the swim-up method results in a decrease in apoptotic sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in a decrease in apoptotic sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method result in at least a 2-fold decrease in apoptotic sperm.

Optionally, the method of evaluating sperm health further comprises identifying sperm that can be used in assisted reproductive technologies (ART), wherein the semen sample is processed using separation techniques, including any one or more of density gradient centrifugation (DGC) and the sperm swim-up method, wherein the DGC method and the swim-up method results in at least a 2-fold decrease in apoptotic processed sperm compared to unprocessed sperm.

Optionally, the method of evaluating sperm health further comprises identifying sperm can be used in assisted reproductive technologies (ART), wherein the semen sample is processed using separation techniques, including any one or more of density gradient centrifugation (DGC) and the sperm swim-up method, wherein the DGC method and the swim-up method results in at least a 2.75-fold decrease in apoptotic processed sperm compared to unprocessed sperm.

Optionally, the method of evaluating sperm health further comprises identifying sperm that can be used in assisted reproductive technologies (ART), wherein the semen sample is processed using separation techniques, such as density gradient centrifugation (DGC) and the sperm swim-up method, wherein the DGC method results in sperm with an increase in MMP.

Optionally, the method of evaluating sperm health further comprises identifying sperm that can be used in assisted reproductive technologies (ART), wherein the semen sample is processed using separation techniques, such as density gradient centrifugation (DGC) and the sperm swim-up method, wherein the swim-up method results in sperm with an increase in MMP.

Optionally, the method of evaluating sperm health further comprises identifying sperm that can be used in assisted reproductive technologies (ART), wherein the semen sample is processed using separation techniques, such as density gradient centrifugation (DGC) and the sperm swim-up method, wherein the DGC method and the swim-up method results in sperm with an increase in MMP.

Optionally, the method of evaluating sperm health further comprises identifying sperm that can be used in assisted reproductive technologies (ART), wherein the semen sample is processed using separation techniques, such as density gradient centrifugation (DGC) and the sperm swim-up method, wherein the DGC method and the swim-up method results in sperm with at least a 1.5-fold increase in MMP.

Optionally, the method of evaluating sperm health further comprises identifying sperm that can be used in assisted reproductive technologies (ART), wherein the semen sample is processed using separation techniques, such as density gradient centrifugation (DGC) and the sperm swim-up method, wherein the DGC method and the swim-up method results in processed sperm with at least a 1.5-fold increase in MMP compared to unprocessed sperm.

Optionally, the method is a method of diagnosing infertility.

Optionally, the method is a method of diagnosing infertility, wherein poor sperm health is indicative of infertility.

Optionally, in the method of diagnosing infertility, the patient is a mammal.

Optionally, in the method of diagnosing infertility, the patient is non-human.

Optionally, in the method of diagnosing infertility, the patient is non-human, such as bovine, equine, canine, feline, porcine, ovine and ursidae animals.

Preferably, in the method of diagnosing infertility, the patient is human.

Optionally, in the method of diagnosing infertility, the patient is non-diabetic human.

Optionally, in the method of diagnosing infertility, the patient is non-diabetic, non-smoking human.

Optionally, in the method of diagnosing infertility, the patient is non-diabetic, non-smoking, non-obese human.

Optionally, in the method of diagnosing infertility, the patient is non-diabetic, non-smoking, non-obese (with a BMI < 29.9) human.

Optionally, in the method of diagnosing infertility, the patient is non-diabetic, non-smoking, non-obese (with a BMI < 29.9) man.

Optionally, the method is a method of diagnosing infertility, the method comprising: providing a biological sample from a patient; measuring the quantitative level of a biomarker in the sample; and diagnosing infertility based on the quantitative level of the biomarker, wherein the biomarker is membrane-bound receptor for advanced glycation end products (mRAGE).

Preferably, the method is a method of diagnosing infertility, the method comprising: providing a semen sample from a patient; measuring the quantitative level of a biomarker in the sample; and diagnosing infertility based on the quantitative level of the biomarker, wherein the biomarker is membrane-bound receptor for advanced glycation end products (mRAGE).

A method involving fertilising an egg is not part of the invention.

### Brief Description of the Drawings

Embodiments of the present invention will now be described with reference to the accompanying drawings and following non-limiting examples, in which:
**Figure 1** illustrates that RAGE is found at the acrosome and equatorial region of the human sperm cell membrane;
**Figure 2** illustrates flow cytometry gating strategy for analysing RAGE protein membrane expression and molecular markers of spermatozoa cell health;
**Figure** 3 illustrates flow cytometry spermatozoa molecular health assay validation;
**Figure 4** illustrates flow cytometry and CASA evaluation of the direct swim-up preparation assay for separating PR motile sperm from washed spermatozoa;
**Figure 5** illustrates RAGE expression follows Gaussian distribution in spermatozoa from a population of non-diabetic, non-smoking and non-obese men;
**Figure** 6 illustrates RAGE is a stably expressed protein on sperm that associates with the immotile sperm population;
**Figure** 7 illustrates flow cytometry quantification of sperm cell health in washed and purified spermatozoa;
**Figure** 8 illustrates RAGE expression on human spermatozoa associates with spermatozoa with low mitochondrial membrane potential, dying and dead sperm with permeable cell membranes;
**Figure** 9 illustrates RAGE expression correlates with the degree of DNA fragmentation in human spermatozoa.
**Figure 10** illustrates evaluation of sperm health and RAGE protein expression in human sperm;
**Figure 11** illustrates evaluation of RAGE protein expression and sperm cell health in unprocessed and processed human sperm;
**Figure 12** illustrates evaluation of correlation between RAGE protein expression and sperm motility after processing via DGC or Swim-Up methods;
**Figure 13** illustrates evaluation of two motility assessment methods and their correlation with sperm Morphology; and
**Figure 14** illustrates evaluation of mRAGE protein expression, sperm health and sperm morphology.

### Examples

### Materials & Methods

### Ethical approval

This study was carried out using semen samples from anonymous patients attending Merrion Fertility Clinic with permission from the donors and ethical approval from the School of Medicine Research Ethics Committee, Trinity College Dublin and the National Maternity Hospital, Holles Street, Ethics Committee. The research was carried out at Trinity Biomedical Sciences Institute, Trinity College Dublin.

### Study population and participants

Men attending the Merrion Fertility Clinic between August 2017 and July 2018 were invited to participate in this project and written consent was received from all participants. The inclusion criteria were samples from normozoospermic participants undergoing a fertility assessment. The exclusion criteria were samples from participants with type I or type II diabetes, smokers and/or individuals with a BMI > 29.9.

### Sample collection and analysis

Semen samples were collected after 2-5 days sexual abstinence. Samples were collected in sterile plastic containers (Starstedt, 75-562-105) and allowed to liquefy for 30 minutes at 37 °C. Semen analysis was performed within 1 h of ejaculation. All samples were subject to conventional analyses for semen volume (ml), sperm concentration (x 10⁶ / ml), sperm morphology and motility, according to World Health Organisation (WHO) recommendations (WHO, 5^{th} Edition).

### Sperm morphology assessment

Sperm morphology assessments were carried out using the simple normal/abnormal classification. Pre-stained slides (TestSimplet, Waldeck) were used following strict Kruger (Tygerberg) criteria. Briefly, a small drop of semen was added to the centre of the coverslip, placed carefully onto the pre-stained area of the slide and incubated for 15 minutes at RT. A small drop of oil was placed on the centre of the coverslip and the 100 X oil immersion lens on an Eclipse E400 microscope was used to assess morphology. Approximately 200 sperm were assessed and the percentage of normal (score > 4% normal) or abnormal (score of < 4% normal) forms were determined.

### Sperm motility assessment

Sperm motility assessments were carried out by CASA using the Hamilton Thorne software and a Zeiss microscope, following WHO and manufacturers' guidelines. Briefly, semen samples were resuspended using Pasteur pipettes and diluted 1 in 3 in sperm wash media. A volume of 3 µl of diluted semen was added to the CASA microscope slide, placed onto the pre-heated stage (37 °C) and allowed to settle until no drift was apparent on the screen. Motility assessment was evaluated by capturing at least 1000 sperm cell tracks per slide for unprocessed samples and 300 for processed samples. Total cell count (x 10⁶/ml), percentage immotile (IM), non-progressively motile (NP) and progressively motile (PR) sperm and average path velocity (VAP) for sperm was recorded for each sample.

### Sperm washing

Seminal plasma was removed by washing spermatozoa into 0.5 ml of sperm wash media (Irvine Scientific, 9983) by centrifugation at 1,500 x g for 1 minute at RT, repeated twice. The final sperm pellet was resuspended in 0.1 ml sperm wash media prior to downstream experiments. The sample is hereafter referred to as "washed spermatozoa".

### Sperm preparation

Semen samples were purified by density gradient centrifugation (DGC) or by direct swim-up. For DGC, the puresperm 40/80 Nidacon PS40-100/PS80-100 separation media as used. A volume of 1.5 ml of 40% lower layer separation media was added to a 15 ml falcon tube followed by 1.5 ml of 80% upper layer separation media. A minimum volume of 1 ml of the semen sample was loaded onto the gradient and centrifuged at 400 x g for 20 minutes at RT. Approximately 2.5 ml of the upper layer was gently removed using a sterile Pasteur pipette and the sperm pellet was washed twice with 2 ml sperm wash media, centrifuged at 380 x g for 5 minutes at RT. The washed pellet, containing highly motile purified sperm, was resuspended in 0.5 ml of sperm wash media. For direct swim-up, a minimum of 1 ml of seminal plasma was washed twice in 2 ml sperm wash media by centrifugation at 380 x g for 5 minutes at RT. The washed sperm pellet was gently resuspended and layered underneath a 1 ml volume of multipurpose handling media (Irvine Scientific, 90166, pre-equilibrated at 37 °C and incubated for 30 minutes at RT. Approximately 0.8 ml of the top layer was removed containing highly motile purified sperm.

### Quantitative assessment of sperm cell health

Measurement of mitochondrial membrane potential (MMP), cell permeability and cell death were determined using MitoTracker CMX Ros (ThermoFisher), DAPI (4',6-diamidino-2-phenylindole; ThermoFisher) and Annexin V-PeCy7 (ThermoFisher), respectively. Briefly, 1 x 10⁶ sperm from seminal plasma were washed into 0.5 ml of sperm wash media followed by a two-step wash into 0.5 ml PBS with 1% FBS. MMP was evaluated by incubation with 6.25 nM Mitotracker CMX Ros (Invitrogen; M7512) for 15 minutes at 37 °C. Cell permeability was evaluated by incubation with 35 ng/ml DAPI (Invitrogen, D1306) for 5 minutes at 37 °C in 0.1 ml PBS with 1% FBS, protected from light. Samples were washed twice with 0.5 ml of PBS with 1% FBS prior to flow cytometry analysis. 1 x 10⁶ sperm were washed into 0.5 ml of 1X Annexin V binding buffer prior to evaluating apoptosis and necrosis using 5 µl of Annexin V-PE-Cy7 (Invitrogen; 8007-72) in 0.1 ml 1X binding buffer and incubating for 15 minutes at RT.

### Quantitative assessment of RAGE protein expression

Measurement of RAGE protein expression was determined using a monoclonal mouse anti-human RAGE antibody-AF647 (Santa Cruz; 80652). Briefly, 1 x 10⁶ spermatozoa were washed into PBS with 1% FBS prior to incubating with 2 µg/ml of RAGE antibody for 1 hour at 37 °C, protected from light. Samples were washed twice with 0.5 ml of PBS with 1% FBS prior to flow cytometry analysis.

### Quantitative assessment of DNA fragmentation

Measurement of DNA fragmentation was determined using the ApoBrdU-terminal deoxynucleotidyl transferase dUTP nick-end labelling (TUNEL) assay, following manufacturer's guidelines (Invitrogen; A23210) with minor modifications. Briefly, after washing in PBS with 1% FBS, 5 x 10⁶ washed sperm cells were fixed with 4% PFA for 30 minutes and washed twice using PBS with 1% FBS. Fixed cells were permeabilised by incubating in 0.1 ml 0.1% Triton-X, 0.1% sodium citrate in PBS for 2 minutes on ice and washed twice with 0.5 ml of wash buffer, supplied with the kit. Fixed and permeabilised cells were incubated with a TdT TUNEL reaction (TdT enzyme, BrdU, reaction buffer) for 1 hour at 37 °C, washed twice with 0.5 ml of rinse buffer and incubated with the mouse anti-BrdU-AF488 antibody (Invitrogen) for 30 mins at RT. Cells were washed twice with 0.5 ml PBS with 1% FBS and counterstained with 10 µl of PI in RNaseA buffer, supplied with the kit.

### Analysis of sperm cell health using flow cytometry

For flow cytometry data collection, a minimum of 10,000 single events were recorded for each sample at a flow rate of 200 events/s using the BD LSR Fortessa flow cytometer. DAPI was measured using the 450/50 band pass filter, CMX Ros using the 610/20 filter, PE-Cy7 using the 780/60 filter, AF647 using the 670/14 filter and AF488 using the 530/30 filter. Spermatozoa were gated using FSC-A and SSC-A in the flame-shaped region to remove debris and cells other than spermatozoa. Doublets were omitted using the FSC-A and FSC-W dot-plot. Each fluorophore was selected and gated against FSC-A with rectangle gates used to select negative and positive populations. A quadrant plot was used to analyse apoptotic and necrotic sperm cells.

### Confocal analysis of RAGE and mitochondrial localization

For microscopic evaluation of RAGE and mitochondrial localisation on sperm, slides were viewed using a Leica SP8 Gated STED confocal microscope. For RAGE localisation analysis, 1 x 10⁶ washed unprocessed human sperm cells were incubated with 4 µg/ml mouse anti-human RAGE primary antibody (Santa Cruz; sc-80652) for 1 hour at 37 °C followed by 2 µg/ml goat anti-mouse AF647 secondary antibody (ThermoFisher; A28181) for 30 minutes at 37 °C. For mitochondrial localisation, 1 x 10⁶ purified human sperm cells (*via* DGC) were incubated with 0.5 µM MitoTracker CMX Ros for 15 minutes at 37 °C. Stained cells were fixed with 0.5 ml 4% PFA pH 7.2 for 20 minutes at RT and incubated with 70 ng/ml DAPI. Slides were prepared by adding 20 µl of stained sperm cells onto poly-L-lysine microscope slides and air-dried at 37 °C prior to adding mounting medium and a coverslip.

### Statistical analysis

Statistical analyses were performed using Prism 6.0 software (GraphPad). Morphology and mRAGE protein expression were compared using parametric unpaired t-test. Motility and RAGE protein expression were compared using parametric paired t-test. Flow cytometry markers were compared using parametric paired t-test. All data was assumed to be normally distributed. Values are expressed as ± SD. A P value of < 0.05 is considered as statistically significant. The Pearson's correlation test was performed to analyse the correlation between RAGE protein expression and motility. A one-way ANOVA test, with a 2-tailed P value, was used to compare flow cytometry marker expression across unprocessed and processed samples. A P value of < 0.05 is considered as statistically significant.

### Example 1

### mRAGE is found at the acrosome and equatorial membrane regions of human sperm cells

RAGE exists in numerous forms (membrane-bound, soluble or secretory), but detection of membrane bound RAGE (mRAGE) on human spermatozoa was central to this study.

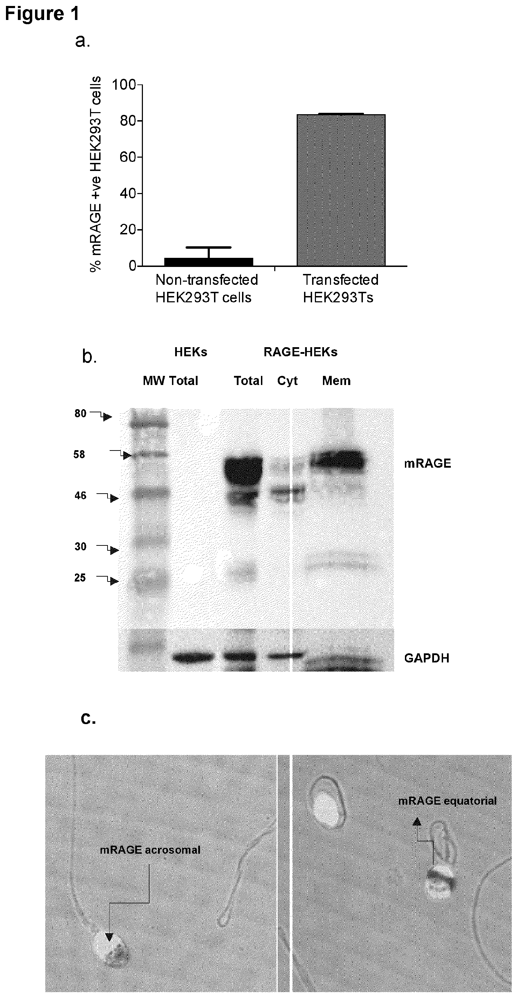

Figure. 1 (a) is a bar chart showing mRAGE positive fluorescence in non-transfected and transfected HEK293T cells analysed with monoclonal RAGE antibody using flow cytometry (n = 2 biological replicates, SD). Figure. 1 (b) is a Western blot analysis of RAGE expression in total, cytosolic and membrane protein lysates from transfected and non-transfected HEK293T cells using a monoclonal mouse anti-human RAGE primary antibody and a mouse anti-HRP secondary antibody. Figure. 1 (c) is a greyscale rendered confocal image showing mRAGE localisation at the acrosome and equatorial region on human sperm cells.

In summary, using a transiently transfected HEK293T cell model, a number of antibodies were screened for mRAGE detection using flow cytometry (Figure. 1 (a)) and mRAGE detection was demonstrated using Western blot (Figure. 1 (b)). The mouse anti-human mRAGE antibody was also capable of detecting mRAGE at the equatorial mid-piece and acrosomal region of the sperm cell head (Figure.1 (c)).

### Example 2

**Flow cytometry gating strategy for analysing mRAGE protein expression and molecular markers of spermatozoa cell health, and flow cytometry spermatozoa molecular health assay validation.**

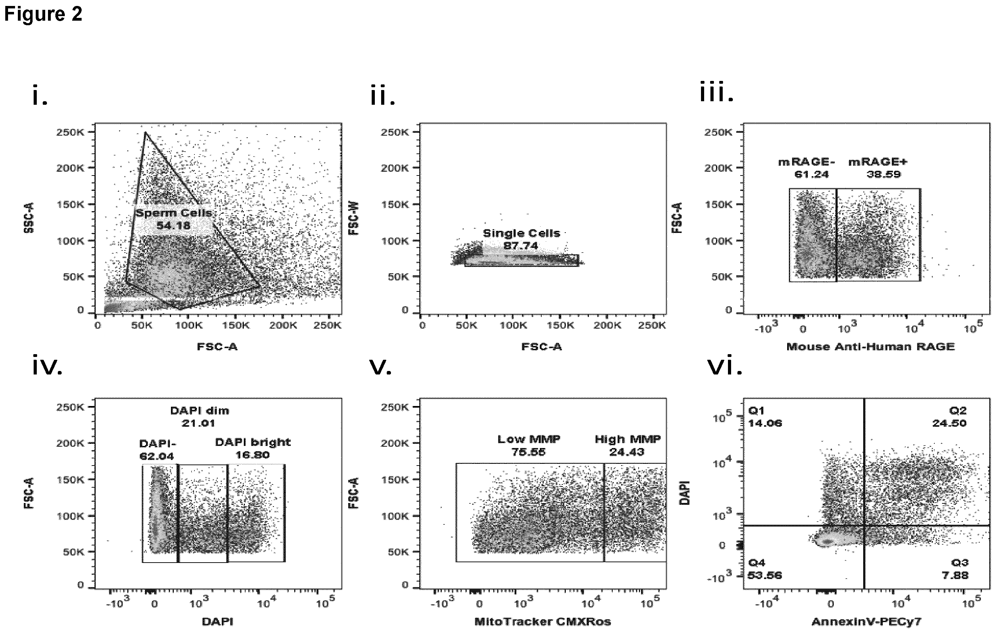

Figure. 2 shows dot plots of the gating strategy for analysing mRAGE protein expression and sperm health in human sperm samples using Flow Cytometry: (i) Polygonal gate drawn around sperm cell population. (ii) Rectangle gate drawn around single cells omitting doublets. (iii) Rectangle gates drawn around mRAGE positive and negative single cell population. (iv) Rectangle gates drawn around DAPI negative, dim and bright single cell population. (v) Rectangle gates drawn around MitoTracker positive (high MMP) and negative (low MMP) single cell population. (vi) Quadrant plots analyzing apoptosis and necrosis: Q1:AnV-DAPI+ (Necrotic); Q2:AnV+DAPI+ (Apoptotic); Q3:AnV+DAPI-(Early apoptotic); Q4:AnV-DAPI- (live).

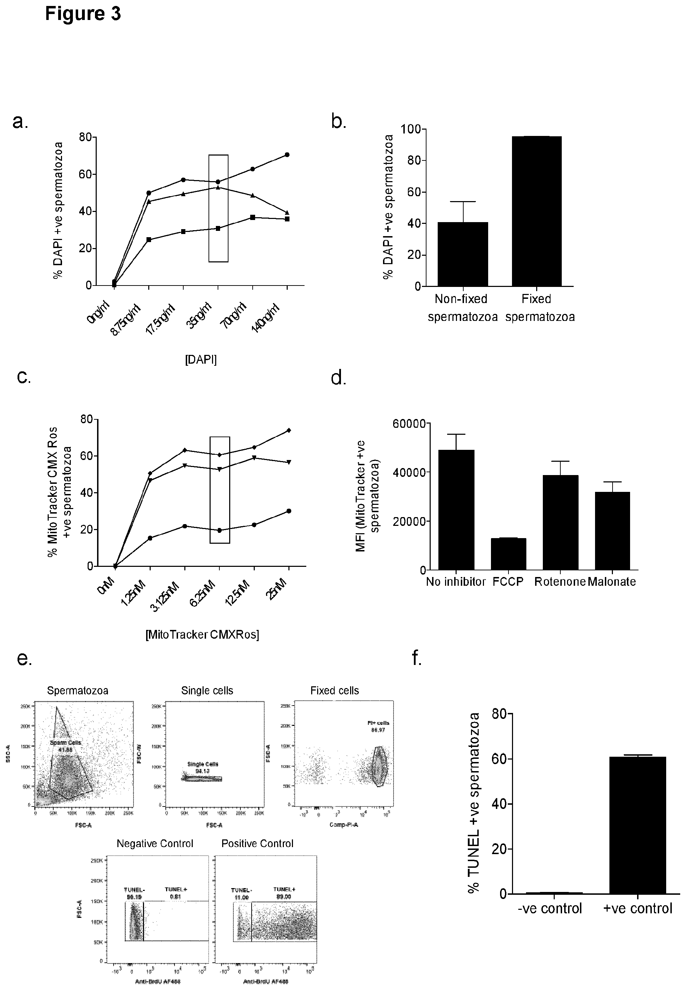

Figure. 3 shows flow cytometry spermatozoa molecular health assay validation. Figure. 3 (a) is a line graph showing human spermatozoa incubated with increasing concentrations of DAPI (n = 3). Final concentration selected for further studies is indicated by the boxed region. Figure. 3 (b) is a bar chart showing nuclear staining in non-fixed and fixed human spermatozoa incubated with 35 ng/ml DAPI. Figure. 3 (c) is a line graph showing human spermatozoa incubated with increasing concentrations of MitoTracker CMX Ros (n = 3). Final concentration selected for further studies is indicated by the boxed region. Figure. 3 (d) is a bar chart showing mean fluorescent intensity (MFI) of MitoTracker CMX Ros positive human spermatozoa in the presence or absence of mitochondrial electron transport chain (ETC) inhibitors; FCCP, Rotenone and Malonate. Figure. 3 (e) is a representative dot plot showing human spermatozoa gating strategy for TUNEL assay (top panels) and the corresponding flow cytometry dot plots for TUNEL negative and positive control reactions (bottom panels) as indicated. Figure. 3 (f) is a bar chart showing TUNEL positive spermatozoa in negative (-TdT enzyme) and positive (+DNasel) control reactions for analysis of DNA fragmentation in human spermatozoa

### Example 3

**Flow cytometry and CASA evaluation of the direct swim-up preparation assay for separating PR motile sperm from washed spermatozoa.**

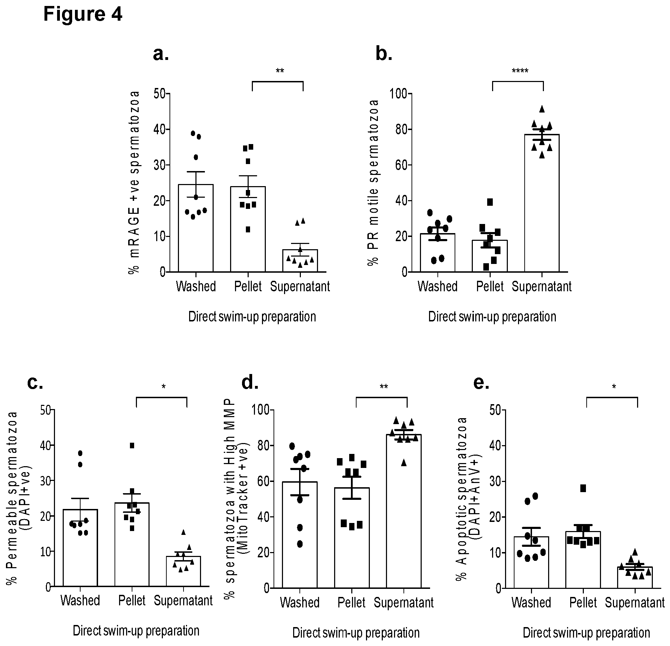

Flow cytometry and CASA evaluation of the direct swim-up preparation assay for separating PR motile sperm from washed spermatozoa. Comparison of percentages of: Figure. 4 (a) mRAGE protein expression, Figure. 4 (b) PR motility, Figure. 4 (c) cell permeability, Figure. 4 (d) MMP and Figure 4. (e) apoptosis in washed spermatozoa versus the pellet and supernatant samples obtained during a direct swim-up preparation (n = 8; % mean ± SEM). One-way ANOVA **** P < 0.0001, ** = P < 0.005; * = P < 0.05.

### Example 4

**mRAGE expression follows Gaussian distribution in spermatozoa from a population of non-diabetic, non-smoking and non-obese men.**

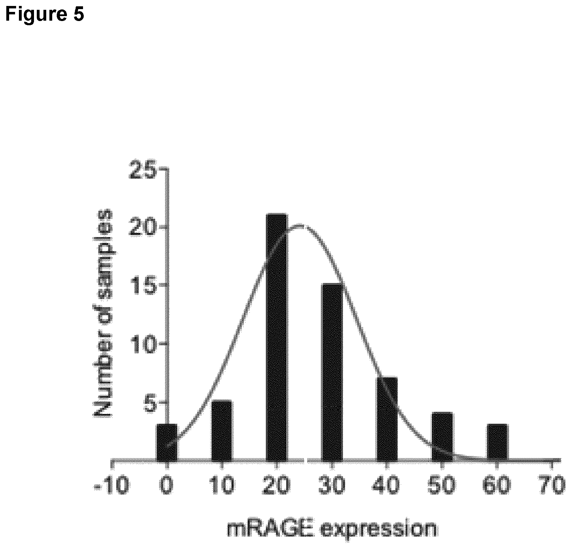

Figure. 5 shows a histogram of frequency distribution of mRAGE expression in n = 60 washed sperm samples measured by flow cytometry.

Overall, it was found that mRAGE expression on human spermatozoa is normally distributed in the population of patients attending fertility clinics in the absence of medical or 'self-inflicted' conditions such as diabetes, smoking or obesity.

### Example 5

**mRAGE is a stably expressed protein on sperm that associates with the immotile sperm population.**

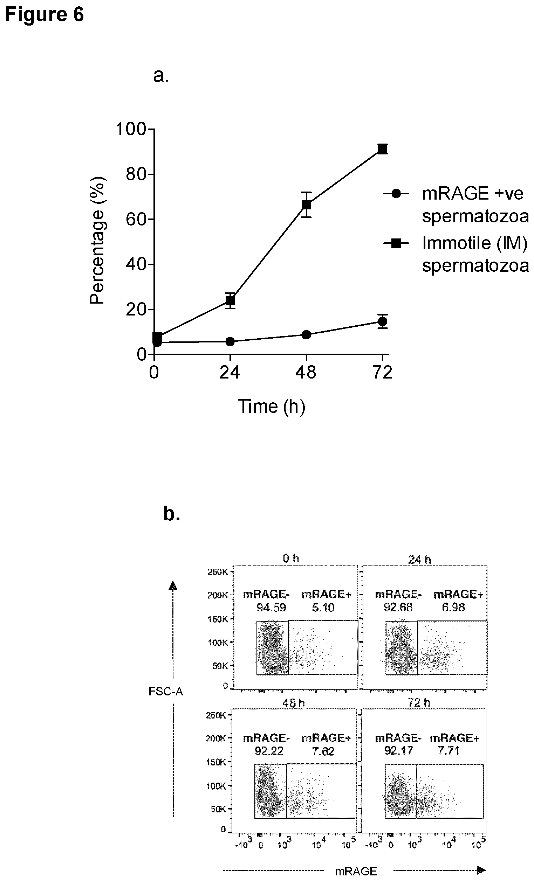

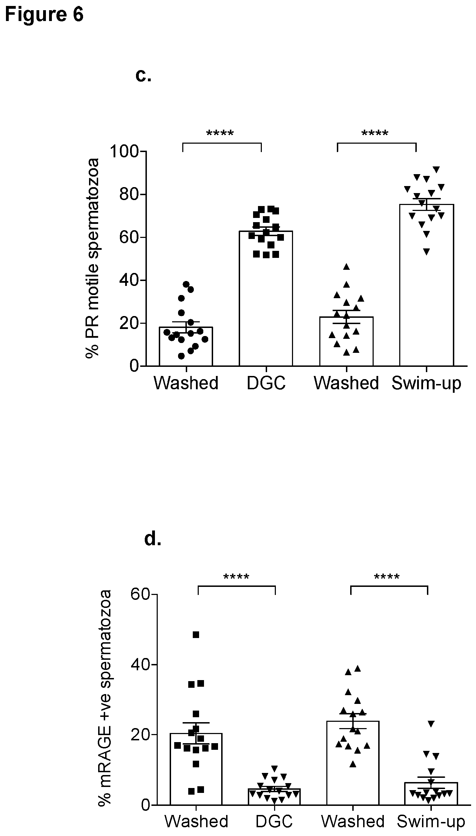

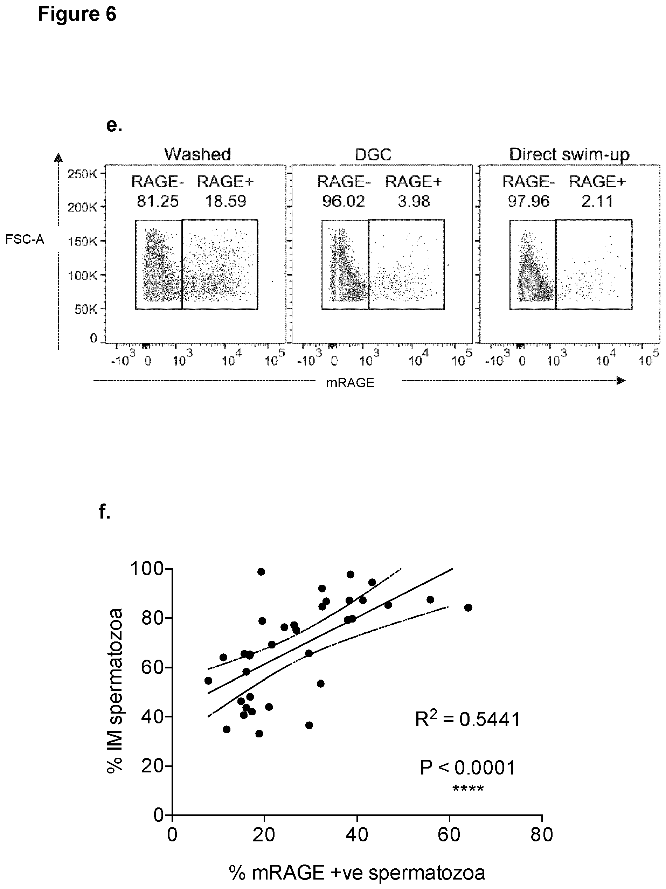

Figure. 6 (a) is a line-graph showing percentage immotile sperm and mRAGE expression in DGC-purified spermatozoa measured over time (0 - 72 h) analysed using CASA and FC (n = 6 % mean ± SEM). Figure. 6 (b) is a dot plot showing mRAGE expression in DGC-purified spermatozoa over time (0 - 72 h). Figure. 6 (c) is a comparison of the percentages of PR motility in washed and purified sperm samples (n = 15; % mean ± SEM). One-way ANOVA; **** = P < 0.0001. Figure. 6 (d) is a comparison of the percentages of mRAGE protein expression in washed and purified sperm samples (n = 15; % mean ± SEM). One-way ANOVA; **** = P < 0.0001. Figure. 6 (e) is a dot plot showing mRAGE expression in washed, DGC and direct swim-up purified sperm samples. Figure. 6 (f) is evaluation of the correlation between % total IM spermatozoa and the percentage of mRAGE protein expression in washed spermatozoa (n = 35). Pearson's correlation R² value indicated on graph; error bars within 95% CI shown by dashed line.

Using a time-course assay, it was observed that mRAGE expression does not differ greatly (4.6 % ± 1.3 (SD) to 14.7 % ± 7.3) over time whereas the percentage of IM sperm in purified samples increases from 7.7 ± 1.9 % (SD) to 91.3 ± 5.1 % (SD) over 72 hours (Figure. 6 (a) and (b), P < 0.0001). This would suggest that mRAGE expression on sperm is directly related to the health status of the individual and does not change following collection of the semen sample. Specifically, the percentage of PR sperm increased from an average of 20.5 ± 10.9 % (SD) in washed samples to 69.1 ± 11.9 % (Figure. 6 (c), P < 0.0001) in purified samples, for both techniques. In contrast, the percentage of mRAGE expressing spermatozoa decreased from an average of 22.1 ± 10.1 % (SD) in washed samples to 5.5 ± 4.8 % (SD) in purified samples (Figure.6 (d) and (e), P < 0.0001). In a larger cohort of samples, it was also observed that mRAGE expression correlates with poor sperm motility (Figure. 6 (f), P < 0.0001) which suggests that mRAGE expression could be used to distinguish between normal and abnormal sperm at a cellular level. This data shows that the absence of mRAGE in the purified samples correlates with PR sperm and improved cell health.

### Example 6

**Flow cytometry quantification of sperm cell health in washed and purified spermatozoa.**

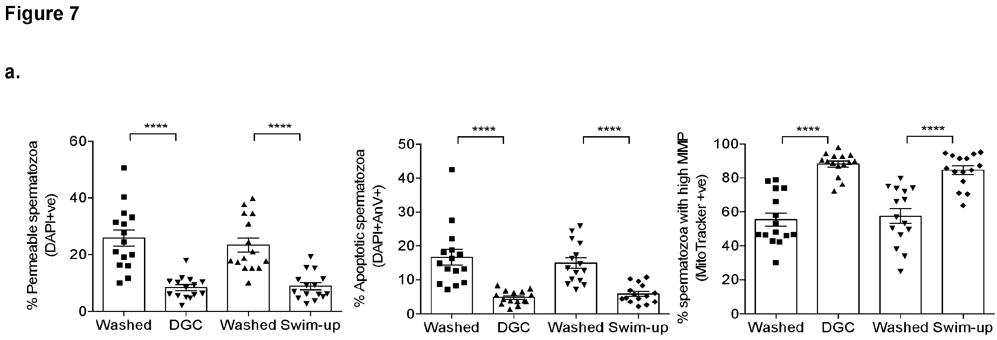

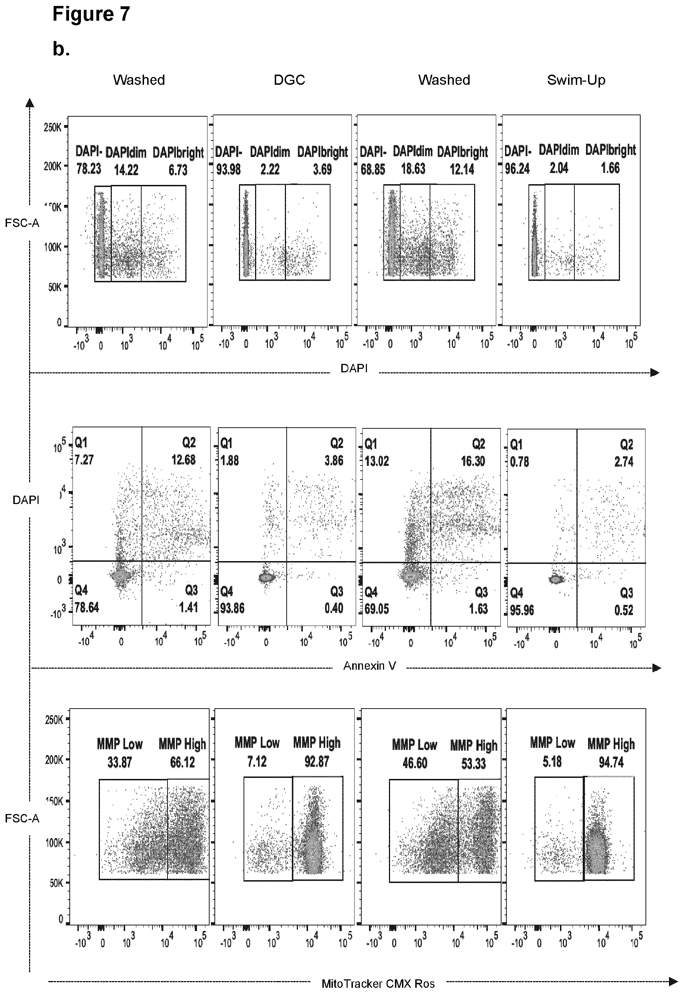

Figure. 7 (a) shows comparison of the percentages of cell permeability, apoptotic cells and MMP in washed and DGC and swim-up preparations (n = 15; % mean ± SEM). One-way ANOVA; **** = P<0.0001. Figure. 7 (b) shows typical dot plots showing DAPI, MitoTracker CMXRos and AnnexinV fluorescence in washed spermatozoa and DGC and swim-up preparations.

Concurrent analysis of the molecular parameters of spermatozoa cell health using flow cytometry revealed that mitochondrial function, the levels of cell death (*via* apoptosis and necrosis) and membrane integrity of spermatozoa greatly improved in the purified samples compared to washed samples (Figure. 7, P < 0.0001).

### Example 7

**mRAGE expression on human spermatozoa associates with spermatozoa with low mitochondrial membrane potential, dying and dead sperm with permeable cell membranes.**

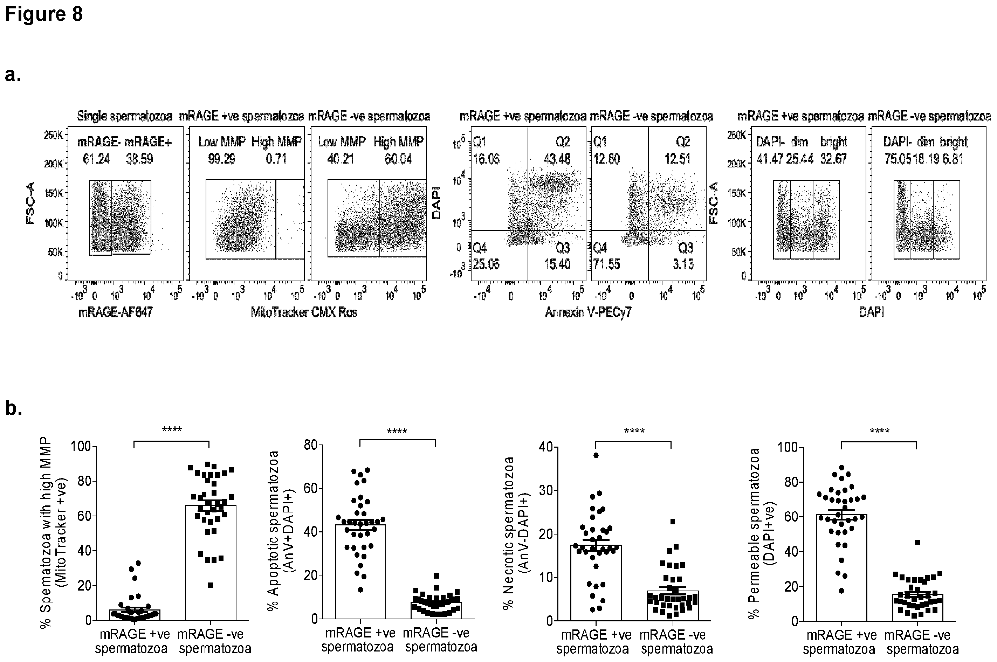

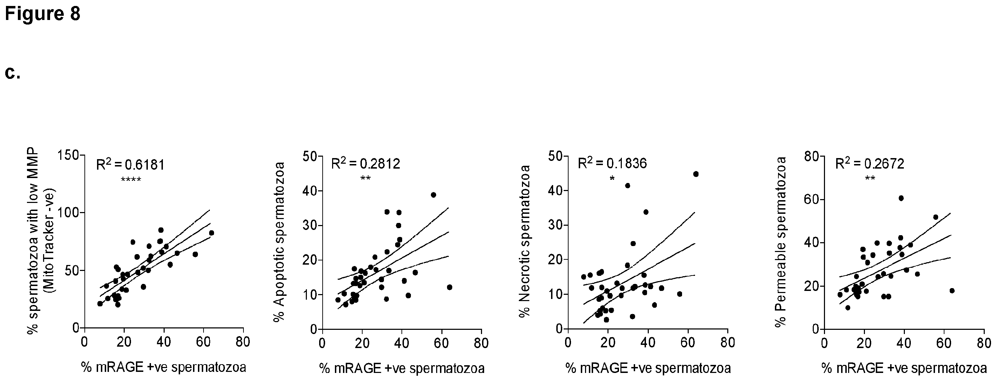

Figure. 8 (a) shows typical dot plots showing mRAGE positive and negative subgroups in washed spermatozoa and MitoTracker (MMP), AnnexinV-DAPI (apoptosis or necrosis) and DAPI (cell permeability) fluorescence in mRAGE positive and negative spermatozoa subgroups. Figure. 8 (b) shows comparison of the percentages of MitoTracker positive, AnnexinV-DAPI positive or negative and DAPI positive spermatozoa in mRAGE positive and negative spermatozoa subgroups (n = 35; % mean ± SEM). Paired t-test; **** = P < 0.0001. Figure. 8 (c) shows evaluation of the correlation between markers of sperm cell health and mRAGE protein expression in washed samples (n = 35). Pearson's correlation R² value indicated on graph; error bars within 95% CI shown by dashed line. **** = P < 0.0001; ** = P < 0.005; * = P < 0.05.

DNA integrity, mitochondrial function and levels of cell death have all been used to analyse molecular health of sperm. Data above showed an improvement to cell health and reduction in mRAGE expressing sperm in purified samples. In a larger cohort of samples, using flow cytometry, it was observed that mRAGE expression on spermatozoa is associated with unhealthy sperm shown by decreased mitochondrial function (10-fold), elevated levels of apoptosis (6-fold) and necrosis (2.5-fold) and decreased membrane integrity (3-fold) in mRAGE positive populations compared to mRAGE negative populations (Figure. 8, P < 0.0001). This data supports the use of mRAGE as a diagnostic biomarker of unhealthy sperm.

To summarise, the data generated confirms that mRAGE protein expression is higher in (i) dead and dying cells, (ii) sperm cells with low mitochondrial membrane potential, (iii) sperm cells dying by apoptosis and (iv) sperm cells dying by necrosis. It would suggest that the mRAGE membrane receptor represents a cell surface marker of an unhealthy sperm cell.

### Example 8

**mRAGE expression correlates with the degree of DNA fragmentation in human spermatozoa.**

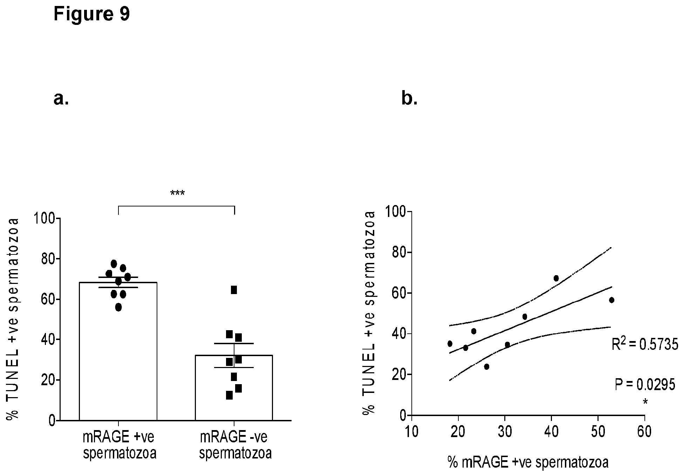

Figure. 9 (a) shows comparison of the percentages of TUNEL positive spermatozoa in mRAGE positive and negative subgroups (n = 8; % mean ± SEM). Paired t-test; *** = P < 0.005. Figure. 9 (b) shows evaluation of the correlation between the percentages of TUNEL positive and mRAGE positive spermatozoa (n = 8). Pearson's correlation R² value indicated; error bars within 95% CI shown by dashed line.

It was observed that the levels of DNA fragmentation, analysed using the TUNEL assay, were significantly higher (2.5-fold) in mRAGE positive spermatozoa compared tom RAGE negative spermatozoa (Figure. 9 (a), P < 0.001) in non-diabetic men. In addition, the data shows that mRAGE expression on sperm correlates with sperm DNA damage (Figure. 9 (b), *P* < 0.05) and could be used as an indirect marker of DNA integrity and sperm cell health.

### Example 9

**Evaluation of sperm health and mRAGE protein expression in human sperm.**

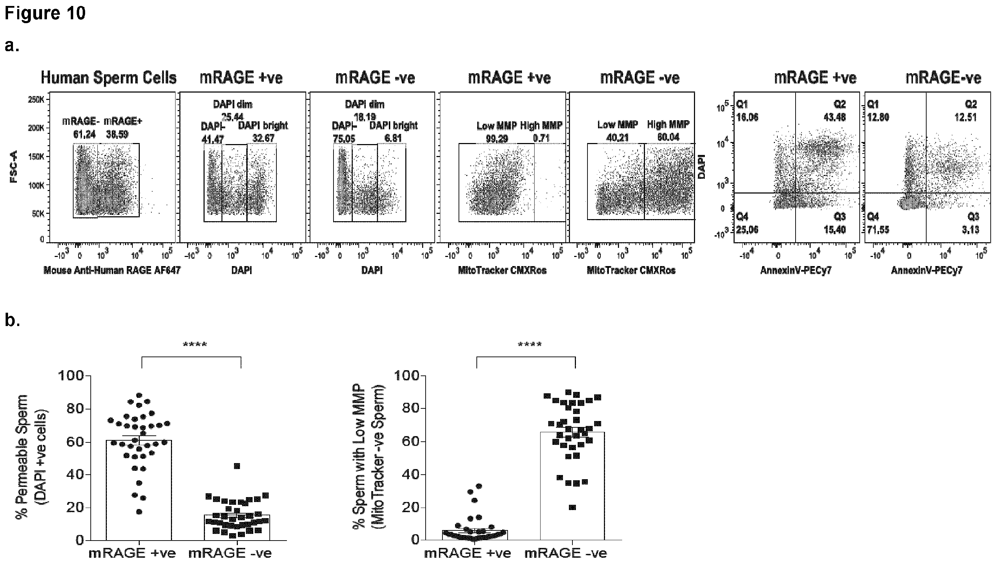

Evaluation of sperm cell health in mRAGE positive and mRAGE negative cell populations in unprocessed sperm. mRAGE protein expression was evaluated using the Mouse Anti-Human mRAGE AF647 antibody; cell permeability was evaluated using DAPI; MMP was evaluated using MitoTracker CMX Ros; apoptosis/necrosis was evaluated using AnnexinV and DAPI. Flow cytometry data was obtained using the LSR Fortessa flow cytometer. Figure. 10 (a) is representative dot plots (n=1) showing mRAGE positive and negative sperm populations with corresponding DAPI, MitoTracker and Annexin V-stained sperm sub-populations. Figure. 10 (b) is evaluation of cell permeability (DAPI positive cells), and MMP (cells with Low MMP) in RAGE positive and negative sperm populations (n=35; % Mean±SEM). Paired t-test; **** = P<0.0001. Figure . 10 (c) is evaluation of apoptotic, early apoptotic and necrotic sperm cells in RAGE positive and negative sperm populations (n=35; % Mean±SEM). Paired t-test; **** = P<0.0001.

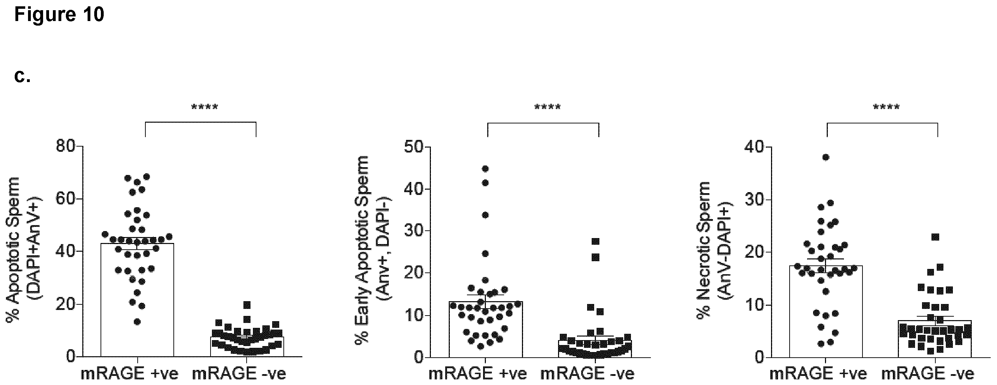

There was a clear distinction between cell permeability, MMP and apoptosis and necrosis in mRAGE positive and negative cell populations when analysing the flow cytometry dot plots (Figure. 10 (a)). Statistical analysis revealed a 3-fold increase in DAPI staining, representing non-viable cells was found in mRAGE positive cell population (Figure. 10 (b), P<0.0001) compared to mRAGE negative cell populations. A 10-fold increase in cells with high MMP was found in mRAGE negative cell populations (Figure. 10 (b), P<0.0001) compared to mRAGE positive cell populations. A 6- and 2.5-fold increase in apoptotic and necrotic cells, respectively, were found in mRAGE positive cell populations compared to mRAGE negative cell populations (Figure. 10 (c), P<0.0001).

### Example 10

**Evaluation of mRAGE protein expression and sperm cell health in unprocessed and processed human sperm.**

The next set of experiments were set up to determine if mRAGE protein expression differed following separation of highly motile sperm from the unprocessed semen sample. Highly motile progressive sperm can be isolated from the total sperm population using the swim-up purification method and/or from DGC purification.

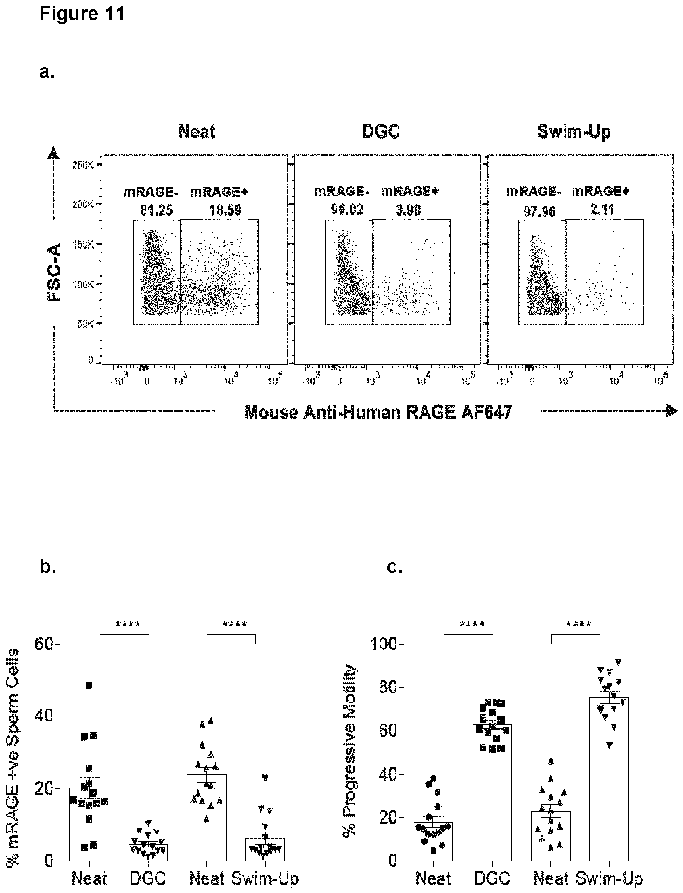

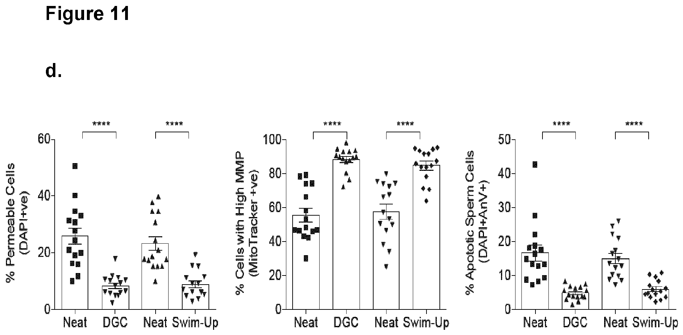

Figure. 11 (a) shows representative dot plots (n=1) showing mRAGE positive and negative sperm populations in unprocessed (Neat) and in sperm processed by DGC and Swim-Up. Figure. 11 (b) shows evaluation of sperm cells expressing mRAGE (mRAGE positive sperm cells) in unprocessed and processed sperm via DGC and Swim-Up (n=15; % MeantSEM). Corresponding unprocessed (neat) fractions for each processing methods included. Paired one-way ANOVA; **** = P<0.0001. Figure. 11 (c) shows evaluation of progressive motility in unprocessed and processed sperm via DGC and Swim-Up (n=15; % Mean±SEM). Corresponding unprocessed (neat) fractions for each processing methods included. Paired one-way ANOVA; **** = P<0.0001. Figure.11 (d) shows evaluation of cell permeability (DAPI positive cells), cells with High MMP and Apoptotic Sperm in unprocessed and processed sperm via DGC and Swim-Up (n=15; % Mean±SEM). Corresponding unprocessed (neat) fractions for each processing methods included. Paired one-way ANOVA; **** = P<0.0001.

A 5-fold and 4-fold decrease in RAGE protein expression (Figure. 11 (b); P<0.0001) was observed in the DGC and Swim-Up purified sperm. In parallel, a 3.5- and 3.4-fold increase in progressively motile sperm (Figure. 11 (c); P<0.0001) in DGC and Swim-Up purified sperm. Alongside this, an average 2.75-fold decrease in impaired cell permeability, 2.75-fold decrease in apoptotic sperm and 1.5-fold increase in MMP (Figure. 11 (d); P<0.0001) was observed in the DGC and swim-up purified sperm.

In summary, sperm purified using both methods discussed here result in highly motile progressive, live sperm that have impermeable cell membranes and highly active mitochondria. In addition, the decrease in mRAGE protein expression in the purified fractions would suggest that mRAGE expressing sperm are removed during the purification process.

### Example 11

**Evaluation of correlation between mRAGE protein expression and sperm motility after processing via DGC or Swim-Up methods.**

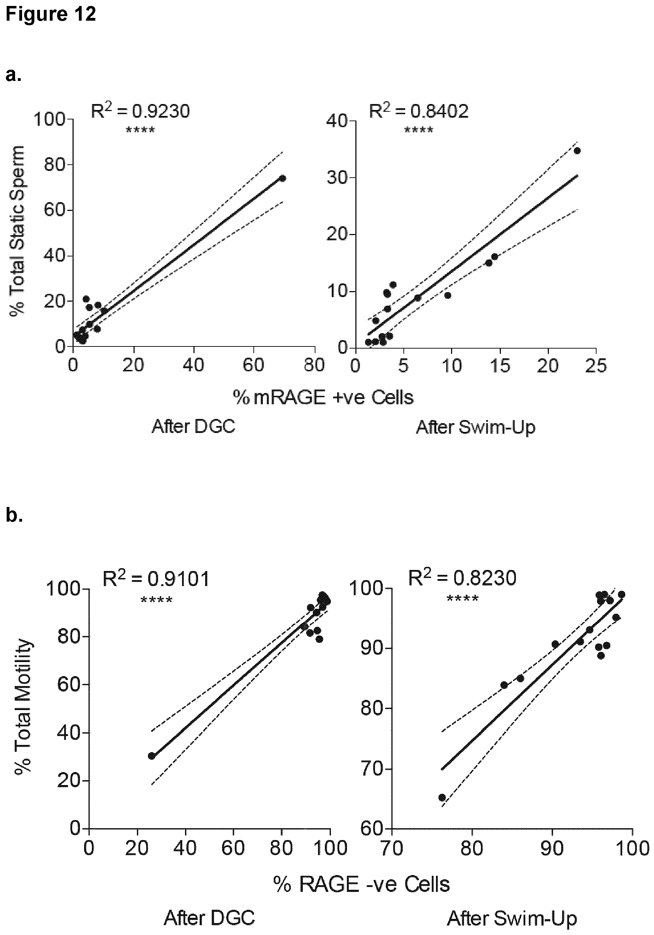

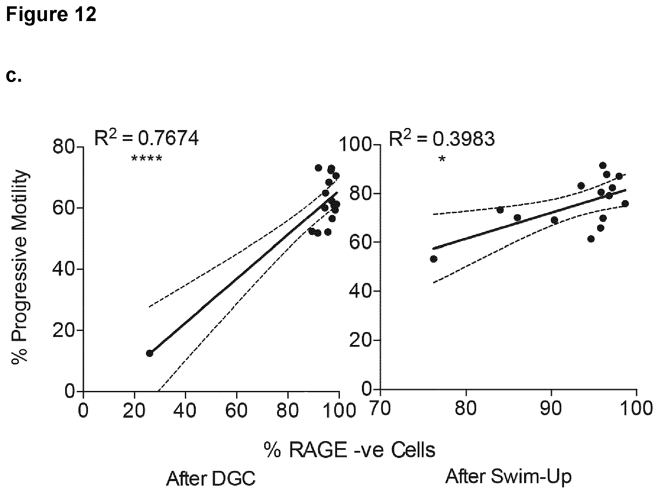

Figure. 12 (a) shows evaluation of the correlation between mRAGE expressing sperm (mRAGE positive cells) and immotile cells present after DGC and Swim-Up processing methods (n=15 % Mean±SEM). Figure. 12 (b) shows evaluation of the correlation between sperm not expressing mRAGE (mRAGE negative cells) and motile sperm present after DGC and Swim-Up processing methods (n=15). Figure.12 (c) shows evaluation of the correlation between sperm not expressing RAGE (RAGE negative cells) and progressive sperm present after DGC and Swim-Up processing methods (n=15). Pearson's correlation R² value indicated on graph; error bars within 95% CI shown by dashed line. **** = P<0.0001.

Further analysis of the data, whereby correlation plots were generated analysing mRAGE protein expression and sperm motility of processed samples, revealed statistically significant correlations between mRAGE protein expression and sperm motility. The statistically significant correlations were observed between mRAGE positive cells and immotile sperm (Figure. 12 (a); R² >0.84), mRAGE negative cells and motile sperm (Figure 12 (b); R² >0.82) and mRAGE negative cells and progressively motile sperm (Figure. 12 (c); R² >0.39).

This data confirms that both Swim-Up and DGC purification methods result in a highly progressive sperm fraction with minimal mRAGE expressing sperm. Any residual mRAGE expressing sperm within the purified fractions could be associated with the small percentages of static/slow sperm remaining after the processing event.

In conclusion, the results from these experiments indicate that mRAGE protein expression correlates with static sperm, whereas conversely, sperm cells not expressing mRAGE are highly motile healthy sperm.

### Example 12

**Evaluation of two motility assessment methods and their correlation with sperm morphology.**

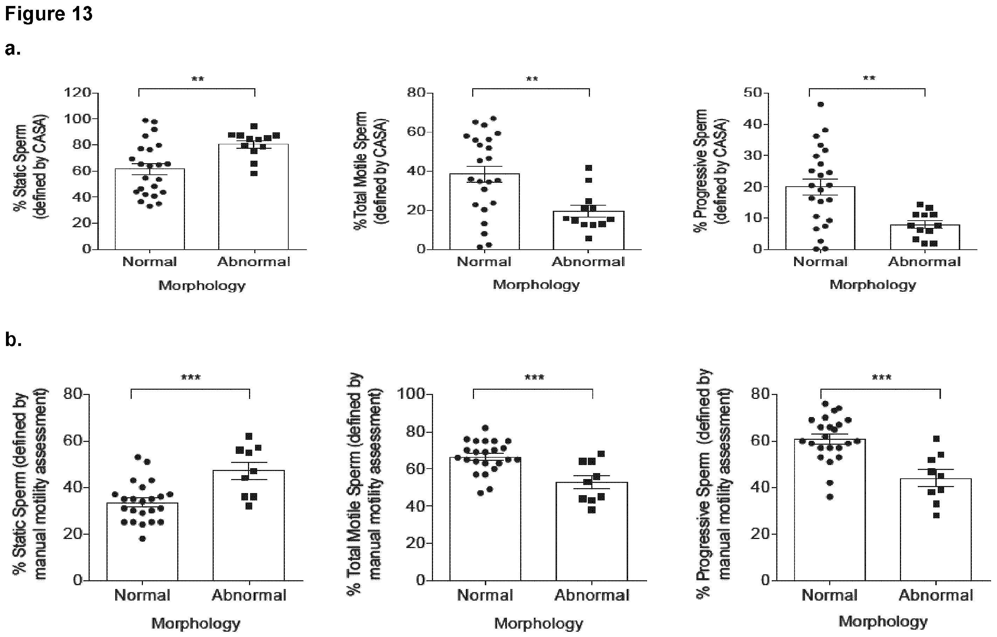

Evaluation of two methods of sperm motility assessments - CASA and manual motility assessment and the correlation with normal VS abnormal morphology in unprocessed semen samples. Sperm morphology was evaluated using Test-Simplet slides and strict Kruger criteria. CASA motility assessment was carried out using the Hamilton Thorne CASA software. Sperm manual motility assessment and morphology assessment was carried out at Merrion Fertility Clinic as part of their routine semen analysis procedures. Figure. 13 (a) Evaluation of sperm motility (static sperm, motile sperm, progressive sperm) measured via CASA in patients with normal and abnormal morphology (n=35; Mean SEM). Unpaired t-test; *** = P<0.0005; ** = P<0.005. Figure. 13 (b) Evaluation of sperm motility (static sperm, motile sperm, progressive sperm) measured via manual motility assessment in patients with normal and abnormal morphology (n=35; Mean SEM). Unpaired t-test; *** = P<0.0005.

A statistically significant increase in static motility measured using CASA (Figure. 13 (a)) and manual methods (Figure. 13 (b)) was observed in sperm samples with abnormal morphology (P = 0.0048 and P = 0.0007, respectively). Parallel results were observed when comparing total motility and progressive motility with sperm morphology (Figure. 13 (a) and Figure. 13 (b); P values ranging from 0.0030-0.007). There is a difference in the statistical significance between the two motility assessment methods, however the trend between the two groups is similar. This data proves the already known phenomenon that motility and morphology correlate with one another and are indicators of sperm health.

### Example 13

### Evaluation of RAGE protein expression, sperm health and sperm morphology

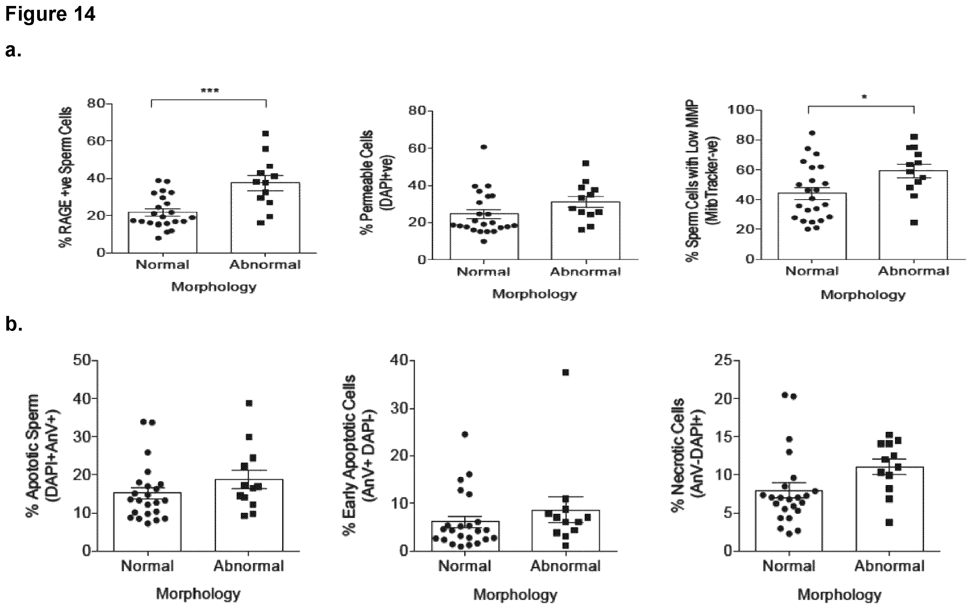

Figure. 14 (a) shows the evaluation of RAGE protein expression, cell permeability (DAPI positive cells), and MMP (cells with low MMP) in patients with normal and abnormal morphology (n=35; % MeantSEM). Unpaired t-test; *** = P<0.0005. * = P<0.05. Figure. 14 (b) shows the evaluation of apoptotic, early apoptotic and necrotic sperm cells in patients with normal and abnormal morphology (n=35; % MeantSEM).

Subsequent analysis of the flow cytometry data and morphology data, carried out using paired t-tests, revealed a statistically significant difference between RAGE protein expression and MMP (Figure. 14 (a); P=0.0002 and P=0.0236, respectively) in patients with normal and abnormal morphology. This data complements the previously determined data and further confirms our hypothesis that mRAGE protein expression is associated with unhealthy, static sperm and now revealing a new relationship with morphologically abnormal sperm.

### Example 14

### Quantifying mRAGE positive and mRAGE negative sperm cells, and Mitochondrial Membrane Potential (MMP)

Table. 1 shows neat samples from processed vs unprocessed experiments (N=15). Mean Fluorescent Intensity (MFI; geometric mean) was analysed in sperm cells that are mRAGE positive vs mRAGE negative, and MFI was analysed in sperm cells with High MMP vs low MMP.

Table. 1 shows the percentage of sperm cells that are mRAGE and mRAGE. Also shown is the MFI (geometric mean) of the sperm cells that are mRAGE positive and mRAGE negative. Also shown is the fold difference between the MFI of mRAGE positive vs mRAGE negative sperm cells. The average fold difference in MFI between mRAGE positive vs mRAGE negative sperm cells is 21.1% +/- 13.8%

Table. 1 shows the percentage of sperm cells with high MMP and low MMP. Also shown is the MFI (geometric mean) of the sperm cells that have high MMP vs low MMP. Also shown s the fold difference between the MFI of sperm cells with high MMP and low MMP. The average fold difference in MFI between cells with high vs low MMP is 25.3% +/- 10.1%.

**Table.1. Analysis of quantifying mRAGE positive and mRAGE negative sperm cells, and Mitochondrial Membrane Potential (MMP)**

| | | **RAGE fluorophore - 640** | | **MMP fluorophore - 540** | | | | | | | |
|---|---|---|---|---|---|---|---|---|---|---|---|
| | | **Percentage sperm cells** | | **MFI (geometric mean)** | | | **Percentage sperm cells** | | **MFI (genometic mean)** | | |
| **Sample #** | **Sample ID** | **mRAGE +** | **mRAGE -** | **mRAGE +** | **mRAGE -** | **Fold difference** | **High MMP** | **Low MMP** | **High MMP** | **Low MMP** | **Fold difference** |
| 1 | 612 | 18.4 | 81.2 | 3212 | 314 | 10.22 | 49.9 | 49.6 | 48223 | 1337 | 36.06 |
| 2 | 620 | 13.4 | 86.2 | 4832 | 282 | 17.13 | 71.6 | 27.1 | 30876 | 1233 | 25.04 |
| 3 | 621 | 16.4 | 83.1 | 5144 | 231 | 22.26 | 71.6 | 25.4 | 26101 | 1320 | 19.77 |
| 4 | 623 | 25 | 72.8 | 4450 | 220 | 20.22 | 54 | 43.3 | 33965 | 1568 | 21.66 |
| 5 | 624 | 16 | 82.8 | 4195 | 214 | 19.60 | 56.4 | 40.7 | 25131 | 999 | 25.15 |
| 6 | 625 | 17.5 | 81.7 | 4436 | 309 | 14.35 | 45.5 | 52.6 | 31103 | 1100 | 28.27 |
| 7 | 626 | 43.9 | 54.9 | 3891 | 277 | 14.04 | 17.1 | 79.1 | 24173 | 1215 | 19.89 |
| 8 | 634 | 22 | 77.4 | 4491 | 313 | 14.34 | 62.3 | 36.4 | 49928 | 3533 | 14.13 |
| 9 | 636 | 25.1 | 74.6 | 5055 | 332 | 15.22 | 45.2 | 53.1 | 75651 | 3697 | 20.46 |
| 10 | 637 | 27.4 | 72.2 | 6189 | 309 | 20.02 | 57.5 | 41.1 | 41706 | 1627 | 25.63 |
| 11 | 658 | 37.6 | 61.4 | 3271 | 379 | 8.63 | 50 | 49.4 | 43358 | 893 | 48.55 |
| 12 | 662 | 59.7 | 39.6 | 3068 | 318 | 9.64 | 14.5 | 81 | 20901 | 518 | 40.34 |
| 13 | 665 | 3.96 | 95.9 | 2991 | 62.4 | 47.93 | 69.7 | 29.4 | 84127 | 5909 | 14.23 |
| 14 | 667 | 3.93 | 95.7 | 1335 | 53 | 25.18 | 45.5 | 53.8 | 36582 | 1740 | 21.02 |
| 15 | 669 | 3.55 | 96.2 | 1951 | 34 | 57.38 | 61.9 | 37 | 32554 | 1671 | 19.48 |
| | **Average** | **22.25** | **77.04** | **3900** | **243** | **21.08** | **51.51** | **46.6** | **40291** | **1890** | **25.31** |
| | **STDEV** | **15.48** | **15.69** | **1275** | **109** | **13.76** | **17.11** | **16.44** | **18308** | **1418** | **10.11** |

## Claims

1. An *in vitro* method of evaluating sperm health; the method comprising the steps of :
(a) providing a biological sample from a patient;
(b) measuring the quantitative or qualitative level of a biomarker in the sample; and
(c) evaluating sperm health based on the quantitative or qualitative level of the biomarker,
wherein the biomarker is membrane-bound receptor for advanced glycation end products (mRAGE).

2. The method according to Claim 1 wherein the evaluating step (c) comprises evaluating the presence or absence of quantifiable levels of mRAGE on sperm cells from the sample to define the patient as mRAGE negative or mRAGE positive.

3. The method according to Claim 2 wherein mRAGE positive is indicative of one or more of poor sperm health, abnormal sperm, poor sperm motility and infertility; and mRAGE negative is indicative of one or more of normal sperm and good sperm health.

4. The method according to Claim 2 or Claim 3 wherein the presence of quantifiable levels of mRAGE on sperm cells from the sample defines the patient as mRAGE positive and wherein the absence of quantifiable levels of mRAGE on sperm cells from the sample defines the patient as mRAGE negative.

5. The method according to any one of Claims 1-4 wherein the evaluating step (c) comprises comparing the amount of sperm expressing quantifiable levels of mRAGE to the amount of sperm not expressing quantifiable levels of mRAGE.

6. The method according Claim 5 wherein the amount of sperm expressing quantifiable levels of mRAGE deviates from the amount of sperm not expressing quantifiable levels of mRAGE by at least 1-fold; optionally wherein deviation of the amount of sperm expressing quantifiable levels of mRAGE from the amount of sperm not expressing quantifiable levels of mRAGE by at least 1-fold defines the patient as mRAGE positive; and/or wherein deviation of the amount of sperm not expressing quantifiable levels of mRAGE from the amount of sperm expressing quantifiable levels of mRAGE by at least 1-fold defines the patient as mRAGE negative.

7. The method according to any of Claims 1-6 wherein a Mean Fluorescent Intensity value of the sperm expressing quantifiable levels of mRAGE deviates from a Mean Fluorescent Intensity value of the sperm not expressing quantifiable levels of mRAGE by at least 10-fold defines the patient as mRAGE positive; and/or wherein a Mean Fluorescent Intensity value of the sperm not expressing quantifiable levels of mRAGE deviates from a Mean Fluorescent Intensity value of the sperm expressing quantifiable levels of mRAGE by at least 10-fold defines the patient as mRAGE negative.

8. The method according to any of Claims 1-7 wherein the method of evaluating sperm health further comprises a step of assessing sperm motility as IM, NP or PR as defined by the World Health Organisation (WHO) laboratory manual for the examination and processing of human semen (fifth edition, 2010), wherein sperm categorised as IM is indicative of poor sperm health; wherein sperm categorised as NP and/or wherein sperm categorised as PR is indicative of good sperm health; optionally wherein the amount of mRAGE positive sperm is inversely correlated to the amount of PR sperm.

9. The method according to any of Claims 1-8 wherein the method of evaluating sperm health further comprises a step of assessing sperm morphology wherein sperm are classified as normal when the sperm head is smooth, regularly contoured and substantially oval in shape; and sperm are classified as abnormal when there is deviation from normal, wherein deviation from normal is indicative of poor sperm health.

10. The method according to any of Claims 1-9 wherein the method of evaluating sperm health further comprises a step of assessing mitochondrial membrane potential (MMP), wherein quantifiable levels of MMP is indicative of mRAGE negative sperm and the absence of quantifiable levels of MMP is indicative of mRAGE positive sperm.

11. The method according to any of Claims 1-10 wherein the method of evaluating sperm health further comprises a step of assessing cell permeability, wherein there is at least a 2-fold decrease in cell permeability in mRAGE positive sperm compared to mRAGE negative sperm is indicative of poor sperm health; and/or wherein the method of evaluating sperm health further comprises a step of assessing apoptosis and necrosis, wherein there is at least a 2-fold increase in apoptosis and necrosis in mRAGE positive sperm compared to mRAGE negative sperm is indicative of poor sperm health; and/or wherein the method of evaluating sperm health further comprises a step of assessing DNA fragmentation, wherein there is at least a 2-fold increase in DNA fragmentation in mRAGE positive sperm compared to mRAGE negative sperm, which is indicative of poor sperm health.

12. The method according to any of Claims 1-11 wherein the method of evaluating sperm health further comprises a step of processing the sample using separation techniques, including any one or more of density gradient centrifugation (DGC) and the sperm swim-up method.

13. The method according to Claim 12 wherein the DGC method and the swim-up method results in:
(a) at least a 2-fold decrease in mRAGE positive sperm compared to mRAGE negative sperm; and/or
(b) at least a 2-fold increase in PR sperm, and/or
(c) sperm with at least a 2-fold decrease in cell permeability, and/or
(d) at least a 2-fold decrease in apoptotic sperm;
which is indicative of sperm that can be used in assisted reproductive technologies (ART) including any one or more of intrauterine insemination (IUI), intracytoplasmic injection (ICSI) and in vitro fertilisation (IVF).

14. The method according to any of Claims 1-13 wherein the patient is any one or more of non-diabetic, non-smoking and non-obese.

15. The method according to any of Claims 1-14 wherein the method is a method of diagnosing infertility.

## Patentansprüche

1. In-vitro-Verfahren zum Beurteilen einer Spermiengesundheit; wobei das Verfahren die folgenden Schritte umfasst:
(a) Bereitstellen einer biologischen Probe von einem Patienten;
(b) Messen des quantitativen oder qualitativen Niveaus eines Biomarkers in der Probe; und
(c) Beurteilen der Spermiengesundheit basierend auf dem quantitativen oder qualitativen Niveau des Biomarkers,
wobei der Biomarker ein membrangebundener Rezeptor für fortgeschrittene Glykationsendprodukte (mRAGE) ist.

2. Verfahren nach Anspruch 1, wobei der Beurteilungsschritt (c) ein Beurteilen des Vorhandenseins oder Nichtvorhandenseins von quantifizierbaren Niveaus von mRAGE auf Spermazellen aus der Probe umfasst, um den Patienten als mRAGE-negativ oder mRAGE-positiv zu definieren.

3. Verfahren nach Anspruch 2, wobei mRAGE-positiv auf eines oder mehrere von einer schlechten Spermiengesundheit, anormalen Spermien, einer schlechten Spermienmotilität und einer Unfruchtbarkeit hinweist; und mRAGE-negativ auf eines oder mehrere von normalen Spermien und einer guten Spermiengesundheit hinweist.

4. Verfahren nach Anspruch 2 oder Anspruch 3, wobei das Vorhandensein von quantifizierbaren Niveaus von mRAGE auf Spermazellen aus der Probe den Patienten als mRAGE-positiv definiert und wobei das Nichtvorhandensein von quantifizierbaren Niveaus von mRAGE auf Spermazellen aus der Probe den Patienten als mRAGE-negativ definiert.

5. Verfahren nach einem der Ansprüche 1 bis 4, wobei der Beurteilungsschritt (c) ein Vergleichen der Menge von Spermien, die quantifizierbare Niveaus von mRAGE exprimieren, mit der Menge von Spermien, die keine quantifizierbaren Niveaus von mRAGE exprimieren, umfasst.

6. Verfahren nach Anspruch 5, wobei die Menge der Spermien, die quantifizierbare Niveaus von mRAGE exprimieren, von der Menge der Spermien, die keine quantifizierbaren Niveaus von mRAGE exprimieren, um mindestens das 1-Fache abweicht; wobei optional eine Abweichung der Menge der Spermien, die quantifizierbare Niveaus von mRAGE exprimieren, von der Menge der Spermien, die keine quantifizierbaren Niveaus von mRAGE exprimieren, um mindestens das 1-Fache den Patienten als mRAGE-positiv definiert; und/oder wobei eine Abweichung der Menge der Spermien, die keine quantifizierbaren Niveaus von mRAGE exprimieren, von der Menge der Spermien, die quantifizierbare Niveaus von mRAGE exprimieren, um mindestens das 1-Fache den Patienten als mRAGE-negativ definiert.

7. Verfahren nach einem der Ansprüche 1 bis 6, wobei ein Abweichen eines mittleren Fluoreszenzintensitätswerts der Spermien, die quantifizierbare Niveaus von mRAGE exprimieren, von einem mittleren Fluoreszenzintensitätswert der Spermien, die keine quantifizierbaren Niveaus von mRAGE exprimieren, um mindestens das 10-Fache den Patienten als mRAGE-positiv definiert; und/oder wobei ein Abweichen eines mittleren Fluoreszenzintensitätswerts der Spermien, die keine quantifizierbaren Niveaus von mRAGE exprimieren, von einem mittleren Fluoreszenzintensitätswert der Spermien, die quantifizierbare Niveaus von mRAGE exprimieren, um mindestens das 10-Fache den Patienten als mRAGE-negativ definiert.

8. Verfahren nach einem der Ansprüche 1 bis 7, wobei das Verfahren zum Beurteilen der Spermiengesundheit ferner einen Schritt zum Bewerten der Spermienmotilität als IM, NP oder PR, wie durch das Weltgesundheitsorganisations(WHO)-Laborhandbuch zur Untersuchung und Aufarbeitung des menschlichen Ejakulates (fünfte Auflage, 2010) definiert, umfasst, wobei als IM kategorisierte Spermien auf eine schlechte Spermiengesundheit hinweisen; wobei als NP kategorisierte Spermien und/oder wobei als PR kategorisierte Spermien auf eine gute Spermiengesundheit hinweisen; wobei optional die Menge der mRAGE-positiven Spermien umgekehrt mit der Menge der PR-Spermien korreliert ist.

9. Verfahren nach einem der Ansprüche 1 bis 8, wobei das Verfahren zum Beurteilen der Spermiengesundheit ferner einen Schritt zum Bewerten einer Spermienmorphologie umfasst, wobei Spermien als normal klassifiziert werden, wenn der Spermienkopf glatt, regelmäßig konturiert und im Wesentlichen oval geformt ist; und Spermien als anormal klassifiziert werden, wenn eine Abweichung von normal vorliegt, wobei eine Abweichung von normal auf eine schlechte Spermiengesundheit hinweist.

10. Verfahren nach einem der Ansprüche 1 bis 9, wobei das Verfahren zum Beurteilen der Spermiengesundheit ferner einen Schritt zum Bewerten eines mitochondrialen Membranpotenzials (MMP) umfasst, wobei quantifizierbare Niveaus von MMP auf mRAGEnegative Spermien hinweisen und das Nichtvorhandensein von quantifizierbaren Niveaus von MMP auf mRAGE-positive Spermien hinweist.

11. Verfahren nach einem der Ansprüche 1 bis 10, wobei das Verfahren zum Beurteilen der Spermiengesundheit ferner einen Schritt zum Bewerten einer Zellpermeabilität umfasst, wobei ein Vorliegen einer mindestens 2-fachen Abnahme der Zellpermeabilität in mRAGE-positiven Spermien verglichen mit mRAGE-negativen Spermien auf eine schlechte Spermiengesundheit hinweist; und/oder wobei das Verfahren zum Beurteilen der Spermiengesundheit ferner einen Schritt zum Bewerten einer Apoptose und Nekrose umfasst, wobei ein Vorliegen einer mindestens 2-fachen Zunahme der Apoptose und Nekrose in mRAGE-positiven Spermien verglichen mit mRAGE-negativen Spermien auf eine schlechte Spermiengesundheit hinweist; und/oder wobei das Verfahren zum Beurteilen der Spermiengesundheit ferner einen Schritt zum Bewerten einer DNA-Fragmentierung umfasst, wobei eine mindestens 2-fache Zunahme der DNA-Fragmentierung in mRAGE-positiven Spermien verglichen mit mRAGE-negativen Spermien vorliegt, was auf eine schlechte Spermiengesundheit hinweist.

12. Verfahren nach einem der Ansprüche 1 bis 11, wobei das Verfahren zum Beurteilen der Spermiengesundheit ferner einen Schritt zum Aufarbeiten der Probe mithilfe von Trenntechniken einschließlich eines oder mehrerer von einer Dichtegradientenzentrifugation (DGC) und eines Spermienaufschwimmverfahrens umfasst.

13. Verfahren nach Anspruch 12, wobei das DGC-Verfahren und das Aufschwimmverfahren zu Folgendem führen:
(a) einer mindestens 2-fachen Abnahme der mRAGE-positiven Spermien verglichen mit mRAGE-negativen Spermien; und/oder
(b) einer mindestens 2-fache Zunahme der PR-Spermien und/oder
(c) Spermien mit einer mindestens 2-fachen Abnahme der Zellpermeabilität und/oder
(d) einer mindestens 2-fachen Abnahme von apoptotischen Spermien;
was auf Spermien hinweist, die bei unterstützten Reproduktionstechnologien (ART) einschließlich eines oder mehrerer von einer intrauterinen Insemination (IUI), einer intrazytoplasmatischen Injektion (ICSI) und einer In-vitro-Fertilisation (IVF) verwendet werden können.

14. Verfahren nach einem der Ansprüche 1 bis 13, wobei der Patient eines oder mehrere von einem Nicht-Diabetiker, einem Nicht-Raucher und einem Nicht-Fettleibigen ist.

15. Verfahren nach einem der Ansprüche 1 bis 14, wobei das Verfahren ein Verfahren zum Diagnostizieren von Unfruchtbarkeit ist.

## Revendications

1. Procédé *in vitro* d'évaluation de la santé des spermatozoïdes ; le procédé comprenant les étapes de :
(a) fourniture d'un échantillon biologique provenant d'un patient ;
(b) mesure du niveau quantitatif ou qualitatif d'un biomarqueur dans l'échantillon ; et
(c) évaluation de la santé des spermatozoïdes sur la base du niveau quantitatif ou qualitatif du biomarqueur,
dans lequel le biomarqueur est un récepteur membranaire pour des produits finaux de glycation avancée (mRAGE).

2. Procédé selon la revendication 1, dans lequel l'étape d'évaluation (c) comprend l'évaluation de la présence ou de l'absence de niveaux quantifiables de mRAGE sur des cellules spermatiques provenant de l'échantillon pour définir le patient comme mRAGE négatif ou mRAGE positif.

3. Procédé selon la revendication 2, dans lequel mRAGE positif indique l'un ou plusieurs d'une mauvaise santé des spermatozoïdes, des spermatozoïdes anormaux, d'une faible mobilité des spermatozoïdes et de l'infertilité ; et mRAGE négatif indique l'un ou plusieurs des spermatozoïdes normaux et d'une bonne santé des spermatozoïdes.

4. Procédé selon la revendication 2 ou la revendication 3, dans lequel la présence de niveaux quantifiables de mRAGE sur des cellules spermatiques provenant de l'échantillon définit le patient comme mRAGE positif et dans lequel l'absence de niveaux quantifiables de mRAGE sur des cellules spermatiques provenant de l'échantillon définit le patient comme mRAGE négatif.

5. Procédé selon l'une quelconque des revendications 1 à 4, dans lequel l'étape d'évaluation (c) comprend la comparaison de la quantité de spermatozoïdes exprimant des niveaux quantifiables de mRAGE à la quantité de spermatozoïdes n'exprimant pas de niveaux quantifiables de mRAGE.

6. Procédé selon la revendication 5, dans lequel la quantité de spermatozoïdes exprimant des niveaux quantifiables de mRAGE s'écarte de la quantité de spermatozoïdes n'exprimant pas de niveaux quantifiables de mRAGE d'au moins un facteur 1 ; éventuellement, dans lequel un écart de la quantité de spermatozoïdes exprimant des niveaux quantifiables de mRAGE par rapport à la quantité de spermatozoïdes n'exprimant pas de niveaux quantifiables de mRAGE d'au moins un facteur 1 définit le patient comme mRAGE positif 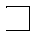 ; et/ou dans lequel un écart de la quantité de spermatozoïdes n'exprimant pas de niveaux quantifiables de mRAGE par rapport à la quantité de spermatozoïdes exprimant des niveaux quantifiables de mRAGE d'au moins un facteur 1 définit le patient comme mRAGE négatif.

7. Procédé selon l'une quelconque des revendications 1 à 6, dans lequel une valeur d'intensité de fluorescence moyenne des spermatozoïdes exprimant des niveaux quantifiables de mRAGE s'écarte d'une valeur d'intensité de fluorescence moyenne des spermatozoïdes n'exprimant pas de niveaux quantifiables de mRAGE d'au moins un facteur 10 définit le patient comme mRAGE positif ; et/ou dans lequel une valeur d'intensité de fluorescence moyenne des spermatozoïdes n'exprimant pas de niveaux quantifiables de mRAGE s'écarte d'une valeur d'intensité de fluorescence moyenne des spermatozoïdes exprimant des niveaux quantifiables de mRAGE d'au moins un facteur 10 définit le patient comme mRAGE négatif.

8. Procédé selon l'une quelconque des revendications 1 à 7, dans lequel le procédé d'évaluation de la santé des spermatozoïdes comprend en outre une étape d'évaluation de la mobilité des spermatozoïdes en tant que IM, NP ou PR, telle que définie par le manuel de laboratoire de l'Organisation mondiale de la santé (OMS) pour l'examen et le traitement du sperme humain (cinquième édition, 2010), dans lequel les spermatozoïdes classés IM indiquent une mauvaise santé des spermatozoïdes ; dans lequel les spermatozoïdes classés NP et/ou dans lequel les spermatozoïdes classés PR indiquent une bonne santé des spermatozoïdes ; éventuellement, dans lequel la quantité de spermatozoïdes mRAGE positifs est inversement corrélée à la quantité de spermatozoïdes PR.

9. Procédé selon l'une quelconque des revendications 1 à 8, dans lequel le procédé d'évaluation de la santé des spermatozoïdes comprend en outre une étape d'évaluation de la morphologie des spermatozoïdes, dans lequel les spermatozoïdes sont classés comme normaux lorsque la tête du spermatozoïde est lisse, de contour régulier et de forme sensiblement ovale ; et les spermatozoïdes sont classés comme anormaux lorsqu'il y a un écart par rapport à la normale, dans lequel un écart par rapport à la normale indique une mauvaise santé des spermatozoïdes.

10. Procédé selon l'une quelconque des revendications 1 à 9, dans lequel le procédé d'évaluation de la santé des spermatozoïdes comprend en outre une étape d'évaluation du potentiel membranaire mitochondrial (MMP), dans lequel des niveaux quantifiables de MMP indiquent des spermatozoïdes mRAGE négatifs et l'absence de niveaux quantifiables de MMP indique des spermatozoïdes mRAGE positifs.

11. Procédé selon l'une quelconque des revendications 1 à 10, dans lequel le procédé d'évaluation de la santé des spermatozoïdes comprend en outre une étape d'évaluation de la perméabilité cellulaire, dans lequel une diminution d'au moins un facteur 2 de la perméabilité cellulaire des spermatozoïdes mRAGE positifs par rapport aux spermatozoïdes mRAGE négatifs indique une mauvaise santé des spermatozoïdes ; et/ou dans lequel le procédé d'évaluation de la santé des spermatozoïdes comprend en outre une étape d'évaluation de l'apoptose et de la nécrose, dans lequel une augmentation d'au moins un facteur 2 de l'apoptose et de la nécrose des spermatozoïdes mRAGE positifs par rapport aux spermatozoïdes mRAGE négatifs indique une mauvaise santé des spermatozoïdes ; et/ou dans lequel le procédé d'évaluation de la santé des spermatozoïdes comprend en outre une étape d'évaluation de la fragmentation de l'ADN, dans lequel une augmentation d'au moins un facteur 2 de la fragmentation de l'ADN dans des spermatozoïdes mRAGE positifs par rapport à des spermatozoïdes mRAGE négatifs indique une mauvaise santé des spermatozoïdes.

12. Procédé selon l'une quelconque des revendications 1 à 11, dans lequel le procédé d'évaluation de la santé des spermatozoïdes comprend en outre une étape de traitement de l'échantillon à l'aide de techniques de séparation, notamment l'une ou plusieurs de la centrifugation en gradient de densité (DGC) et de la méthode de nage ascendante des spermatozoïdes.

13. Procédé selon la revendication 12, dans lequel la méthode DGC et la méthode de nage ascendante donnent :
(a) une diminution d'au moins un facteur 2 des spermatozoïdes mRAGE positifs par rapport aux spermatozoïdes mRAGE négatifs ; et/ou
(b) une augmentation d'au moins un facteur 2 des spermatozoïdes PR, et/ou
(c) des spermatozoïdes présentant une diminution d'au moins un facteur 2 de la perméabilité cellulaire, et/ou
(d) une diminution d'au moins un facteur 2 des spermatozoïdes apoptotiques ;
ce qui indique des spermatozoïdes pouvant être utilisés dans les techniques de procréation assistée (TPA), notamment l'une ou plusieurs de l'insémination intra-utérine (IIU), de l'injection intracytoplasmique (ICSI) et de la fécondation in vitro (FIV).

14. Procédé selon l'une quelconque des revendications 1 à 13, dans lequel le patient est non diabétique et/ou non fumeur et/ou non obèse.

15. Procédé selon l'une quelconque des revendications 1 à 14, dans lequel le procédé est un procédé de diagnostic de l'infertilité.
